# EUROPÄISCHE PATENTANMELDUNG

(11) **EP 2 033 666 A2**
(43) Veröffentlichungstag der Anmeldung: **11.03.2009**
(21) Anmeldenummer: 08165943.5
(22) Anmeldetag: 28.05.2004
(51) Int. Cl.: A61L 27/30, A61L 27/56, A61L 31/08, A61L 31/14

(54) **Implantate mit funktionalisierten Kohlenstoffoberflächen**

(30) Priorität: 28.05.2003 DE 10324415; 21.07.2003 DE 10333098; 21.07.2003 DE 10333099
(62) Teilanmeldung aus: 04735213.3
(71) Anmelder: CINVENTION AG, 65203 Wiesbaden (DE)
(72) Erfinder: Rathenow, Jörg, 49610 Quakenbrück (DE); Asgari, Sohéil, 65193 Wiesbaden (DE); Bán, Andreas, 40764 Langenfeld (DE); Kunstmann, Jürgen, 65812 Bad Soden (DE); Mayer, Bernhard, 63071 Offenbach am Main (DE)
(74) Vertreter: Maiwald Patentanwalts GmbH

(57) **Zusammenfassung**

Die vorliegende Erfindung betrifft ein Verfahren zur Herstellung medizinischer Implantate mit funktionalisierten Oberflächen, durch Bereitstellung eines medizinischen Implantats mit mindestens einer kohlenstoffhaltigen Schicht auf mindestens einem Teil der Oberfläche des Implantats, Aktivierung der kohlenstoffhaltigen Schicht durch Schaffung von Porosität, sowie Funktionalisierung der aktivierten, kohlenstoffhaltigen Schicht, und danach erhältliche funktionalisierte Implantate.

## Beschreibung

Die vorliegende Erfindung betrifft ein Verfahren zur Herstellung medizinischer Implantate mit funktionalisierten Oberflächen, durch Bereitstellung eines medizinischen Implantats mit mindestens einer kohlenstoffhaltigen Schicht auf mindestens einem Teil der Oberfläche des Implantats, Aktivierung der kohlenstoffhaltigen Schicht durch Schaffung von Porosität, sowie Funktionalisierung der aktivierten, kohlenstoffhaltigen Schicht, und danach erhältliche, funktionalisierte Implantate.

Medizinische Implantate wie chirurgische bzw. orthopädische Schrauben, Platten, Gelenkprothesen, künstliche Herzklappen, Gefäßprothesen, Stents als auch subkutan oder intramuskulär implantierbare Wirkstoffdepots werden aus verschiedenartigsten Materialien, die nach den spezifischen biochemischen und mechanischen Eigenschaften ausgewählt werden, hergestellt. Diese Materialien müssen für den dauerhaften Einsatz im Körper geeignet sein, keine toxischen Stoffe freisetzen und bestimmte mechanische und biochemische Eigenschaften aufweisen.

Die beispielsweise für Stents und Gelenkprothesen häufig verwendeten Metalle oder Metalllegierungen, sowie keramischen Materialien weisen jedoch häufig, insbesondere beim Dauereinsatz, Nachteile hinsichtlich ihrer Biokompatibilität oder Funktionalität auf. Implantate lösen durch chemische und/oder physikalische Reizung inflammatorische Gewebe- und Immunreaktionen aus, so dass es zu Unverträglichkeitsreaktionen im Sinne von chronischen Entzündungsreaktionen mit Abwehr- und Abstoßungsreaktionen, überschießender Narbenbildung oder Gewebeabbau kommt, die im Extremfall dazu führen müssen, dass das Implantat entfernt und ersetzt werden muss, oder aber zusätzliche therapeutische Interventionen invasiver oder nichtinvasiver Art angezeigt sind.

Aus diesem Grund gab es im Stand der Technik verschiedene Ansätze die Oberflächen medizinischer Implantate in geeigneter Weise zu beschichten, um die Biokompatibilität der verwendeten Materialien oder die funktionelle Wirksamkeit der Implantate zu erhöhen und Abwehr bzw. Abstoßungsreaktionen zu verhindern.

In der US 5,891,507 werden beispielsweise Verfahren zur Beschichtung der Oberfläche von Metallstents mit Silikon, Polytetrafluorethylen sowie biologischen Materialien wie Heparin oder Wachstumsfaktoren beschrieben, welche die Bioverträglichkeit der Metallstents erhöhen.

Neben Kunststoffschichten haben sich kohlenstoffbasierte Schichten als besonders vorteilhaft erwiesen.

So sind beispielsweise aus der DE 199 51 477 Koronarstents mit einer Beschichtung aus amorphem Siliziumkarbid bekannt, welches die Biokompatibilität des Stentmaterials erhöht. Das US-Patent 6,569,107 beschreibt kohlenstoffbeschichtete Stents, bei welchen das Kohlenstoffmaterial mittels chemischer oder physikalischer Dampfphasenabscheidungs-methoden (CVD oder PVD) aufgebracht wurde. Auch im US-Patent 5,163,958 werden rohrförmige Endoprothesen oder Stents mit einer kohlenstoffbeschichteten Oberfläche beschrieben, die antithrombogene Eigenschaften aufweist. Die WO 02/09791 beschreibt Intravaskularstents mit Beschichtungen die durch CVD von Siloxanen erzeugt werden.

Neben den CVD-Verfahren zur Abscheidung von Kohlenstoff werden im Stand der Technik verschiedene Sputterverfahren im Hochvakuum zur Herstellung pyrolytischer Kohlenstoffschichten mit verschiedener Struktur beschrieben, siehe hierzu beispielsweise die US 6,355,350.

Die so hergestellten Implantate mit modifizierten Oberflächen weisen jedoch einige Nachteile auf. So ist die Biokompatibilität nicht in allen Fällen ausreichend, um Abstoßungsreaktionen vollständig zu verhindern. Ferner sind die oberflächenbeschichteten Implantate des Standes der Technik in der Regel geschlossenporig, was ein Zusammenwachsen mit dem umliegenden Körpergewebe erschwert oder verhindert oder die Funktionalisierung einschränkt. Obwohl diese Implantate des Standes der Technik beispielsweise auch mit Antibiotika beschichtet werden können, ist die Wirkung dieser Stoffe nach Einsetzen des Implantats jedoch stets nur von kurzer Dauer, da die aufgebrachten Mengen des Wirkstoffs durch die Natur des Implantats und seiner Oberflächenbeschichtung begrenzt sind oder deren Desorption nicht kontrollierbar ist oder aber deren Wirksamkeit durch physikalische oder chemische Wechselwirkung mit der Beschichtung beeinträchtigt wird.

Ferner ist es aus medizinischer Sicht sinnvoll und wünschenswert, wenn Implantate nicht nur in ihrer stützenden Funktion, wie bei Stents, verwendet werden können, sondern auch mit zusätzlichen Funktionen versehen werden können, zum Beispiel einer langfristigen Abgabe vor Arzneistoffen am Einsatzort des Implantats, um die Wirkung des Implantats zu verstärken oder aber zusätzliche medizinisch wünschenswerte Wirkungen zu erzeugen.

Es besteht daher ein Bedarf nach einfach anwendbaren und kostengünstigen Verfahren zur Herstellung funktionalisierter Implantate.

Ferner besteht ein Bedarf nach kostengünstig herzustellenden medizinischen Implantaten mit verbesserten Eigenschaften.

Eine Aufgabe der vorliegenden Erfindung ist es daher, ein Verfahren zur Herstellung von Implantaten mit zusätzlicher Funktionalität zur Verfügung zu stellen.

Eine weitere Aufgabe der vorliegenden Erfindung ist es, medizinische Implantate zur Verfügung zu stellen, die zusätzliche Funktionen, wie z. B. die Abgabe von Arzneistoffen im Körper oder die Ansiedlung von Geweben, übernehmen können und dabei erhöhte Bioverträglichkeit bzw. Biokompatibilität aufweisen bzw. eine stärkere funktionelle Implantatwirkung aufweisen.
Eine weitere Aufgabe der vorliegenden Erfindung ist es, medizinische Implantate bereit zu stellen, die eine dauerhafte Freisetzung von medizinischen Wirkstoffen im Körper eines Patienten ermöglichen, oder eine durch Oberflächenmodifikation verbesserte Funktion aufweisen.
Eine wiederum weitere Aufgabe der vorliegenden Erfindung ist es, medizinische Implantate zur Verfügung zu stellen, welche aufgebrachte bzw. inkorporierte pharmakologisch wirksame Stoffe nach dem Einsetzen des Implantats in den menschlichen Körper gezielt und/oder kontrolliert freisetzen können.
Eine weitere Aufgabe der Erfindung ist es, implantierbare Wirkstoffdepots mit einer Beschichtung bereitzustellen, welche die Freisetzung von Wirkstoffen aus dem Depot steuern kann.
Eine weitere Aufgabe der Erfindung ist es, medizinische Implantate zur Verfügung zu stellen, welche aufgebrachte bzw. inkorporierte Mikroorganismen, virale Vektoren oder Zellen oder Gewebe enthalten, so dass nach dem Einsetzen des Implantats in den menschlichen Körper gezielt eine therapeutische Wirkung erzeugt werden kann oder die Bioverträglichkeit gesteigert werden kann.

Die erfindungsgemäße Lösung der oben genannten Aufgaben besteht in einem Verfahren sowie damit erhältlichen medizinischen Implantaten wie in den unabhängigen Ansprüchen definiert. Bevorzugte Ausführungsformen des erfindungsgemäßen Verfahrens bzw. der erfindungsgemäßen Erzeugnisse und Verwendungen ergeben sich aus den abhängigen Unteransprüchen.

Im Rahmen der vorliegenden Erfindung wurde gefunden, dass sich insbesondere kohlenstoffhaltige Schichten auf implantierbaren medizinischen Vorrichtungen unterschiedlichster Art auf einfache Weise dazu nutzen lassen, das Implantat mit zusätzlichen medizinisch-physiologischen und therapeutischen Funktionen auszustatten.

Insbesondere ist es erfindungsgemäß möglich, therapeutisch wirksame Mengen an Arzneistoffen auf der Oberfläche eines Implantats oder in einer auf dem Implantat vorhandenen Schicht zu fixieren und im menschlichen Körper dauerhaft und kontrolliert freizusetzen.

Entsprechend umfasst das erfindungsgemäße Verfahren zur Herstellung medizinischer Implantate mit funktionalisierten Oberflächen die folgenden Schritte:
a) Bereitstellung eines medizinischen Implantats mit mindestens einer kohlenstoffhaltigen Schicht auf mindestens einem Teil der Oberfläche des Implantats;
b) Aktivierung der kohlenstoffhaltigen Schicht durch Schaffung von Porosität;
c) Funktionalisierung der aktivierten, kohlenstoffhaltigen Schicht.

Mit dem erfindungsgemäßen Verfahren lassen sich Implantate mit kohlenstoffhaltigen Oberflächenbeschichtungen geeignet modifizieren, so dass eine Beladung mit therapeutisch wirksamen Mengen an pharmakologisch wirksamen Substanzen möglich ist. Durch Erzeugung von Porosität in kohlenstoffhaltigen Oberflächenschichten geeigneter Stärke, gezielte Einstellung/Modifizierung der Porengröße und/oder Porenstruktur, sowie gegebenenfalls geeignete freisetzungsmodifizierende Oberflächenbeschichtung lassen sich Beladungsmenge, Art und Geschwindigkeit der Freisetzung sowie die biologisch-physiologischen Oberflächeneigenschaften gezielt einstellen und variieren. Auf diese Weise lassen sich maßgeschneiderte Lösungen für jede Art von Implantat und Wirkstoff sowie jeden Anwendungsort und Anwendungszweck der medizinischen Implantate mit einfachen Verfahrensmaßnahmen wie erfindungsgemäß beschrieben realisieren.

### IMPLANTATE

Mit dem erfindungsgemäßen Verfahren können kohlenstofflialtig beschichtete Implantate funktionalisiert werden.

Die Begriffe "implantierbare, medizinische Vorrichtung" und ,Implantat" werden im weiteren synonym verwendet und umfassen medizinische oder therapeutische Implantate wie beispielsweise Gefäßendoprothesen, intraluminale Endoprothesen, Stents, Koronarstents, periphere Stents, chirurgische bzw. orthopädische Implantate für temporäre Zwecke wie chirurgische Schrauben, Platten, Nägel und sonstige Befestigungsmittel, permanente chirurgische oder orthopädische Implantate wie Knochen- oder Gelenkprothesen, beispielsweise künstliche Hüft- oder Kniegelenke, Gelenkpfanneneinsätze, Schrauben, Platten, Nägel, implantierbare orthopädische Fixierungshilfsmittel, Wirbelkörperersatzmittel, sowie Kunstherzen und Teile davon, künstliche Herzklappen, Herzschrittmachergehäuse, Elektroden, subkutane und/oder intramuskulär einsetzbare Implantate, Wirkstoffdepots und Mikrochips, und dergleichen.

Die im erfindungsgemäßen Verfahren verwendbaren Implantate können aus nahezu beliebigen, vorzugsweise im wesentlichen temperaturstabilen Materialien bestehen, insbesondere aus allen Materialien, aus denen typischerweise Implantate hergestellt werden.

Beispiele hierfür sind amorpher und/oder (teil-)kristalliner Kohlenstoff, Vollkarbonmaterial, poröser Kohlenstoff, Graphit, Kohlenstoffverbundmaterialien, Kohlefasern, Keramiken wie z. B. Zeolithe, Silikate, Aluminiumoxide, Aluminosilikate, Siliziumkarbid, Siliziumnitrid; Metallkarbide, Metalloxide, Metallnitride, Metallcarbonitride, Metalloxycarbide, Metalloxynitride und Metalloxycarbonitride der Übergangsmetalle wie Titan, Zirkonium, Hafnium, Vanadium, Niob, Tantal, Chrom, Molybdän, Wolfram, Mangan, Rhenium, Eisen, Kobalt, Nickel; Metalle und Metalllegierungen, insbesondere der Edelmetalle Gold, Silber, Ruthenium, Rhodium, Palladium, Osmium, Iridium, Platin; Metalle und Metalllegierungen von Titan, Zirkon, Hafnium, Vanadin, Niob, Tantal, Chrom, Molybdän, Wolfram, Mangan, Rhenium, Eisen, Kobalt, Nickel, Kupfer; Stahl, insbesondere rostfreier Stahl, Formgedächtnislegierungen wie Nitinol, Nickel-Titanlegierung, Glas, Stein, Glasfasern, Mineralien, natürliche oder synthetische Knochensubstanz, Knochenimitate auf Basis von Erdalkalimetallkarbonaten wie Kalziumkarbonat, Magnesiumkarbonat, Strontiumkarbonat, geschäumte Materialien wie Polymerschäume, geschäumte Keramik und dergleichen sowie beliebige Kombinationen der genannten Materialien.

In bevorzugten Ausführungsformen der vorliegenden Erfindung sind die verwendeten Implantate Stents, insbesondere Metallstents, vorzugsweise aus rostfreiem Stahl, Platinhaltigen radiopaken Stahllegierungen, sogenannten PERSS (platinum enhanced radiopaque stainless steel alloys), Kobaltlegierungen, Titanlegierungen, hochschmelzenden Legierungen beispielsweise auf Basis von Niob, Tantal, Wolfram und Molybdän, Edelmetallegierungen, Nitinollegierungen, sowie Magnesiumlegierungen und Mischungen der Vorgenannten.

Besonders bevorzugte Implantate im Rahmen der vorliegenden Erfindung sind Stents aus rostfreiem Stahl, insbesondere Fe-18Cr-14Ni-2.5Mo ("316LVM" ASTM F138), Fe-21Cr-10Ni-3.5Mn-2.5Mo (ASTM F 1586), Fe-22Cr-13Ni-5Mn (ASTM F 1314), Fe-23Mn-21Cr-1Mo-1N (Nickelfreier rostfreier Stahl); aus Kobaltlegierungen wie z.B. Co-20Cr-15W-10Ni ("L605" ASTM F90), Co-20Cr-35Ni-10Mo ("MP35N" ASTM F 562), Co-20Cr-16Ni-16Fe-7Mo ("Phynox" ASTM F 1058); Beispiele bevorzugter Titanlegierungen sind CP Titanium (ASTM F 67, Grade 1), Ti-6Al-4V (Alpha/beta ASTM F 136), Ti-6Al-7Nb (alpha/beta ASTM F1295), Ti-15Mo (beta grade ASTM F2066); Stents aus Edelmetalllegierungen, insbesondere Iridiumhaltige Legierungen wie Pt- 10Ir; Nitinollegierungen wie martensitische, superelastische und kaltbearbeitete (bevorzugt 40%) Nitinole; sowie Magnesiumlegierungen wie Mg-3A1-1Z.

Die erfindungsgemäß verwendbaren implantierbaren medizinischen Vorrichtungen können nahezu beliebige äußere Formen aufweisen; das erfindungsgemäße Verfahren ist nicht auf bestimmte Strukturen beschränkt.

Die Implantate müssen eine kohlenstoffhaltige Schicht auf zumindest einem Teil ihrer Oberfläche aufweisen. Diese Schicht kann aus pyrolytisch erzeugtem Kohlenstoff, glasartig amorphem Kohlenstoff, aufgedampftem Kohlenstoff, mittels CVD-, PVD- oder Sputtern aufgebrachtem Kohlenstoff, diamantartigem Kohlenstoff, graphitischem Kohlenstoff, Metallcarbiden, Metallcarbonitriden, Metalloxynitriden oder Metalloxycarbiden, sowie beliebigen Kombinationen davon bestehen. Die kohlenstoffhaltige Schicht kann amorph, teilkristallin oder kristallin sein, bevorzugt sind Schichten aus amorphem, pyrolytischem Kohlenstoff, in einigen Ausführungsformen auch diamantartiger, beispielsweise aufgedampfter Kohlenstoff.

Besonders bevorzugt sind kohlenstoffhaltig beschichtete Implantate, die durch Aufbringen Kohlenstoff erzeugender Materialien und/oder polymerer Filme auf das Implantat und anschließende Karbonisierung dieser Materialien unter Sauerstoffausschluss und bei erhöhter Temperatur hergestellt werden. Beispiele hierfür sind in der DE 10322187 bzw. PCT/EP2004/005277, DE 10324415 bzw. PCT/EP2004/004987 oder DE 10333098 bzw. PCT/EP2004/004985 offenbart, deren Offenbarungen hiermit per Zitierung einbezogen werden.

Weitere geeignete kohlenstoffhaltig beschichtete Implantate sind handelsübliche karbonbeschichtete Implantate, wie zum Beispiel Metallstents vom Typ Radix Carbostent® (Sorin Biomedica) und dergleichen, welche meist mittels physikalischer Dampfabscheidungs- oder Zerstäubungsverfahren, auch Sputtern, hergestellte Kohlenstoffbeschichtungen enthalten.

Die Dicke der einen oder mehreren kohlenstoffhaltigen Schicht(en) kann in allgemeinen von 1 nm bis 1 mm, gegebenenfalls auch mehrere Millimeter betragen, z.B. bis zu 10 mm, vorzugsweise bis zu 6 mm, besonders bevorzugt bis zu 2 mm, insbesondere zwischen 10 nm und 200 µm.

In bevorzugten Ausführungsformen der vorliegenden Erfindung können die implantierbaren medizinischen Vorrichtungen auch mehrere kohlenstoffhaltige Schichten gleicher oder unterschiedlicher Dicke und/oder Porosität aufweisen. So lassen sich beispielsweise tieferliegende porösere Schichten mit darüber liegenden engporigen Schichten kombinieren, welche die Abgabe von in der stärker porösen Schicht deponierten Wirkstoffen geeignet verzögern können.

### AKTIVIERUNG

Gemäß dem erfindungsgemäßen Verfahren werden die physikalischen und chemischen Eigenschaften der kohlenstoffbasierten Beschichtung durch geeignete Aktivierungsschritte weiter modifiziert und dem jeweils gewünschten Verwendungszweck angepasst. Herkömmliche kohlenstoffbeschichtete Implantate weisen meist in Wesentlichen geschlossene Oberflächen auf, welche eine wirksame und dauerhafte Beladung mit z.B. Wirkstoffen stark einschränken oder auf sehr geringe Mengen begrenzen. Zweck der Aktivierung ist es, eine Porosität in der kohlenstoffhaltigen Schicht zu schaffen bzw. eine poröse kohlenstoffhaltige Schicht auf dem Implantat zu bilden, um so eine bessere Funktionalisierung mittels Wirkstoffen, Zellen, Proteinen, Mikroorganismen etc. zu ermöglichen und die Aufnahmefähigkeit der kohlenstoffhaltigen Schicht je Flächeneinheit zu erhöhen.

Der Aktivierungsschritt im erfindungsgemäßen Verfahren besteht somit im Wesentlichen darin, Porosität in der Kohlenstoffschicht auf dem Implantat zu erzeugen. Hierfür stehen mehrere Möglichkeiten zur Verfügung.

Eine mögliche Aktivierung der Kohlenstoffschicht umfasst beispielsweise reduzierende oder oxidative Behandlungsschritte, bei welchen die Schicht mit geeigneten Reduktionsmitteln und/oder Oxidationsmitteln wie Wasserstoff, Kohlendioxid, Wasserdampf, Sauerstoff, Luft, Distickstoffmonoxid, oder auch oxidierenden Säuren wie Salpetersäure und dergleichen sowie ggf. Mischungen dieser ein oder mehrfach behandelt wird.

Bevorzugt ist die Aktivierung mit Luft, besonders bevorzugt bei erhöhter Temperatur.

Der oder die Aktivierungsschritte können ggf. bei erhöhter Temperatur, beispielsweise von 40°C bis 1000°C, vorzugsweise 70°C bis 900°C, besonders bevorzugt 100°C bis 850°C, insbesondere bevorzugt 200°C bis 800°C und insbesondere bei etwa 700 °C durchgeführt werden. In besonders bevorzugten Ausführungsformen wird die kohlenstoffhaltige Schicht reduktiv oder oxidativ, oder mit einer Kombination dieser Behandlungsschritte bei Raumtemperatur modifiziert. Auch Kochen in oxidierenden Säuren oder in Laugen kann zur Erzeugung einer porösen Oberfläche verwendet werden.

Je nach Art der verwendeten Oxidations- oder Reduktionsmittel, Temperatur und Dauer der Aktivierung lassen sich Porengröße und Porenstruktur variieren. In besonders bevorzugten Ausführungsformen können erfindungsgemäß aktivierte kohlenstoffbeschichtete medizinische Implantate durch gezielte Einstellung der Porosität der Kohlenstoffschicht zur kontrollierten Abgabe von Wirkstoffen aus dem Substrat in die äußere Umgebung verwendet werden.

Bevorzugt sind die Beschichtungen nach der Aktivierung porös, insbesondere nanoporös. Hierin lassen sich beispielsweise erfindungsgemäße medizinische Implantate als Arzneistoffträger mit Depotwirkung verwenden, insbesondere auch wenn das Implantat selbst zusätzlich eine poröse Struktur aufweist, wobei die aktivierte, kohlenstoffbasierte Schicht des Implantats als freisetzungsregulierende Membran genutzt werden kann.

In bevorzugten Ausführungsformen kann die Einstellung der Porosität durch Herauswaschen von in der kohlenstoffhaltigen Beschichtung vorhandenen Füllstoffen, wie beispielsweise Polyvinylpyrrolidon, Polyethylenglycol, Aluminiumpulver, Fettsäuren, Mikrowachse- oder -Emulsionen, Paraffine, Carbonate, gelöste Gase, oder wasserlösliche Salze mit Wasser, Lösemittel, Säuren oder Laugen oder durch Destillation oder oxidative bzw. nichtoxidative thermische Zersetzung erfolgen. Geeignete Methoden hierzu sind in der DE 103 22 187 bzw. in der PCT/EP2004/005277 desselben Anmelders beschrieben, deren Offenbarung hiermit vollständig per Zitierung einbezogen wird.

Gegebenenfalls kann die Porosität auch durch Strukturierung der Oberfläche mit pulverförmigen Substanzen wie beispielsweise Metallpulver, Ruß, Phenolharzpulver, Fasern, insbesondere Kohlenstoff- oder Naturfasern erzeugt werden.

Eine weitere Möglichkeit der Aktivierung bzw. Porositätserzeugung ist das Besputtern der kohlenstoffhaltigen Schicht mit geeigneten Elementen, oder auch sogenanntes "Ion-Bombarding", beispielsweise mit Edelgasionen oder dergleichen.

Die aktivierte Beschichtung kann gegebenenfalls auch in einem weiteren optionalen Verfahrensschritt, einem sogenannten CVD-Prozeß (Chemical Vapour Deposition, chemische Gasphasenabscheidung) oder CVI-Prozeß (Chemical Vapour Infiltration) unterzogen werden, um die Oberflächen- oder Porenstruktur und deren Eigenschaften weiter zu modifizieren. Hierzu wird die karbonisierte Beschichtung mit geeigneten, kohlenstoffabspaltenden Precursorgasen bei hohen Temperaturen behandelt. Bevorzugt ist hier die nachträgliche Aufbringung von diamantartigem Kohlenstoff. Auch andere Elemente können damit abgeschieden werden, beispielsweise Silizium. Derartige Verfahren sind im Stand der Technik bekannt.

Als kohlenstoffabspaltende Precursor kommen nahezu alle bekannten gesättigten und ungesättigten Kohlenwasserstoffe mit ausreichender Flüchtigkeit unter CVD-Bedingungen in Frage. Beispiele hierfür sind Methan, Ethan, Ethylen, Acetylen, lineare und verzweigte Alkane, Alkene und Alkine mit Kohlenstoffzahlen von C₁ - C₂₀, aromatische Kohlenwasserstoffe wie Benzol, Naphthalin etc., sowie ein- und mehrfach alkyl-, alkenyl- und alkinylsubstituierte Aromaten wie beispielsweise Toluol, Xylol, Cresol, Styrol etc.

Als Keramik-Precursor können BCl₃, NH₃, Silane wie SiH₄, Tetraethoxysilan (TEOS), Dichlorodimethylsilan (DDS), Methyltrichlorosilan (MTS), Trichlorosilyldichloroboran (TDADB), Hexadichloromethylsilyloxid (HDMSO), AlCl₃, TiCl₃ oder Mischungen davon verwendet werden.

Diese Precursor werden in CVD-Verfahren zumeist in geringer Konzentration von etwa 0,5 bis 15 Vol.-% in Mischung mit einem Inertgas, wie beispielsweise Stickstoff, Argon oder dergleichen angewendet. Auch der Zusatz von Wasserstoff zu entsprechenden Abscheidegasgemischen ist möglich. Bei Temperaturen zwischen 500 und 2000°C, vorzugsweise 500 bis 1500°C und besonders bevorzugt 700 bis 1300°C, spalten die genannten Verbindungen Kohlenwasserstofffragmente bzw. Kohlenstoff oder keramische Vorstufen ab, die sich im Porensystem der pyrolysierten Beschichtung im wesentlichen gleichmäßig verteilt niederschlagen, dort die Porenstruktur modifizieren und so zu einer im wesentlichen homogenen Porengröße und Porenverteilung führen.

Mittels CVD-Methoden lassen sich gezielt Poren in der kohlenstoffhaltigen Schicht auf dem Implantat verkleinern, bis hin zur völligen Schließung/Versiegelung der Poren. Hierdurch lassen sich die sorptiven Eigenschaften, wie auch die mechanischen Eigenschaften der aktivierten Implantatoberfläche maßgeschneidert einstellen.

Durch CVD von Silanen oder Siloxanen, gegebenenfalls im Gemisch mit Kohlenwasserstoffen lassen sich die kohlenstoffhaltigen Implantatbeschichtungen durch Carbid- oder Oxycarbidbildung beispielsweise oxidationsbeständig modifizieren.

In bevorzugten Ausführungsformen können die erfindungsgemäß aktivierten, beschichteten Implantate mittels Sputterverfahren zusätzlich beschichtet bzw. modifiziert werden. Hierzu können Kohlenstoff, Silizium oder Metalle bzw. Metallverbindungen aus geeigneter Sputtertargets nach an sich bekannten Verfahren aufgebracht werden. So können durch Einbau von Silizium-, Titan-, Zirkonium- oder Tantalverbindungen oder Metallen mittels CVD oder PVD in die kohlenstoffhaltige Schicht Carbidphasen gebildet werden, welche die Stabilität und Oxidationsbeständigkeit der Schicht erhöhen.

In einer weiteren bevorzugten Ausführungsform des Aktivierungsschritts lassen sich kohlenstoffhaltige Schichten, z.B. auch aufgesputterte C-Schichten, nachträglich mechanisch bearbeiten, um poröse Oberflächen zu erzeugen. So führt beispielsweise die gezielte Abrasion dieser Schichten mittels geeigneter Verfahren zu porösen Schichten. Eine bevorzugte Möglichkeit stellt die Abrasion von solchen kohlenstoffhaltigen Schichten im Ultraschallbad dar, wo durch die Beimengung von abrasiven Feststoffen unterschiedlicher Partikelgrößen und Härtegrade durch angemessenen Energieeintrag und geeigneter Frequenz des Ultraschallbades in Abhängigkeit der Einwirkungszeit gezielte Schichtdefekte und somit Porosität erzeugt werden können.

Bevorzugt sind hierbei wässrige Ultraschallbäder unter Zusatz von Tonerde, Silikaten, Aluminaten und dergleichen, vorzugsweise Tonerdedispersionen. Jedoch können auch beliebige andere für Ultraschallbäder geeignete Lösungsmittel anstelle oder in Mischung mit Wasser verwendet werden.

Beispielsweise lassen sich durch die Behandlung von kohlenstoffbeschichteten Implantaten in einem wässrigen Ultraschallbad unter Beimengung von Tonerde, bevorzugt 1%-ige bis hin zu 60% Tonerde-Dispersionen, nano-abradierte Kohlenstoffschichten mit einer mittleren Porengröße von etwa 5 nm bis 200 nm erhalten.

Ferner können mittels Ionenimplantierung von Metallen, insbesondere Übergangsmetallen, und/oder Nichtmetallen die Oberflächeneigenschaften des Implantats weiter modifiziert werden; so können beispielsweise durch Stickstoffimplantierung Nitride, Oxynitride oder Carbonnitride, insbesondere solche der Übergangsmetalle eingebaut werden. Durch Ionenimplantierung von Kohlenstoff lassen sich Porosität und Festigkeit der Oberflächenmaterialien darüber hinaus weiter modifizieren.

Bevorzugt ist die kohlenstoffhaltige Schicht nach der Aktivierung porös, mit Porendurchmessern im Bereich von 0,1 bis 1000 µm, bevorzugt zwischen 1 µm bis 400 µm. Auch makroporöse Schichten lassen sich mit den erfindungsgemäßen Aktivierungsschritten erzielen.

Besonders bevorzugt ist die kohlenstoffhaltige Schicht nach der Aktivierung nanoporös, mit Porendurchmessern von 1 nm bis 1000 nm, bevorzugt von 5 nm bis 900 nm.

In einer besonders bevorzugten Ausführungsform der Erfindung erfolgt die Aktivierung bereits während des Herstellungsschritts der kohlenstoffhaltigen Schicht, z.B. durch Aufbringung einer oder mehrerer poröser kohlenstoffhaltiger Schichten, durch Karbonisierung kohlenstofferzeugender Substanzen, durch Beschichtung mit Kohlenstoff mittels CVD oder PVD, und/oder durch Aufbringung geeigneter Schichten aus porösen biologisch abbaubaren bzw. resorbierbaren oder nicht-biologisch abbaubaren bzw. resorbierbaren Polymeren.

Besonders bevorzugt ist, dass eine oder mehrere poröse kohlenstoffhaltige Schichten durch Beschichten des Implantats mit einem gegebenenfalls geschäumten oder füllstoffhaltigen Polymerfilm, und anschließendes Karbonisieren des Polymerfilms bei Temperaturen von 200 bis 3500 °C, vorzugsweise bis 2000 °C in sauerstofffreier Atmosphäre aufgebracht werden, die gegebenenfalls nachträglich im Luftstrom teiloxidiert werden können. Entsprechende Verfahren sind beispielsweise in DE 10324415 bzw. PCT/EP2004/004987, oder in DE 10333098 bzw. PCT/EP2004/004985 beschrieben, deren Offenbarung hiermit vollständig miteinbezogen wird.

So führt beispielsweise die Beimischung von Polyethylenglycol in den zu carbonisierenden Polymerfilm zu Defekten in der Polymervernetzung, welche nach thermischer Behandlung oder Herauslösen in geeigneten Lösungsmitteln zu porösen Kohlenstoffschichten führt. Durch die Wahl des Polymersystems, des Molekulargewichts von Polyethylenglycol und des Polyethylenglycol-Feststoffgehalts können der Anwendung entsprechende Porositäten, insbesondere die mittlere Porengröße, die Porengrößenverteilung und der Grad der Porosität eingestellt werden. Beispielsweise können durch die Wahl von Polyethylenglycolen mit einem Molekulargewicht von 1000 bis 8000000 Dalton Porengrößen von 10 bis 1000 nm erzeugt werden, in bevorzugter Ausführungsform von 50 bis 1000 nm. Durch die Variierung des Feststoffgehalts von 10% bis 80% lassen sich Porositätsgrade von 5% bis 80% erzeugen, bevorzugt von 20% bis 60%.

Ein weiteres Beispiel dieser Art der kombinierten Erzeugung und Aktivierung der kohlenstoffhaltigen Schicht stellt die Beimengung von Ruß in den Polymerfilm dar. Durch die Wahl der mittleren Partikelgröße und des Feststoffgehalts im Polymerfilm lassen sich poröse Matrizes herstellen, deren Porositätsgrad und mittlere Porengröße durch die Wahl geeigneter Polymersysteme, Russ-Partikelgrößen und des Feststoffgehalts je nach Anwendung einstellen lässt. So kann beispielsweise durch die Beimengung von Russpartikeln einer mittlerer Partikelgröße von 10 nm bis 1 mm, bevorzugt von 10 nm bis 1000 nm, bei einem Feststoffgehalt von 20 bis 80%, bevorzugt von 30% bis 60%, eine mittlere Porosität von 30-60% erzeugen, wobei die hergestellten Porengrößen zwischen 10 bis 1000 nm, bevorzugt von 10 bis 800 nm einstellbar sind.

Ferner lassen sich durch eine optionale Parylenierung der Implantate vor oder nach dem Aktivierungsschritt Oberflächeneigenschaften und Porosität der kohlenstoffhaltigen Schicht weiter modifizieren. Hierbei werden die Implantate zunächst bei erhöhter Temperatur, üblicherweise etwa 600 °C mit Paracyclophan behandelt, wobei auf den Implantaten oberflächlich ein Polymerfilm aus Poly(p-xylylen) ausgebildet wird. Dieser lässt sich in einem nachfolgenden Karbonisierungsschritt nach bekannten Verfahren in Kohlenstoff umwandeln.

Sofern erforderlich kann in besonders bevorzugten Ausführungsformen das aktivierte Implantat weiteren chemischen und/oder physikalischen Oberflächenmodifikationen unterzogen werden. Auch Reinigungsschritte zur Entfernung von eventuellen Rückständen und Verunreinigungen können hier vorgesehen werden. Hierzu können Säuren, insbesondere oxidierende Säuren, oder Lösemittel verwendet werden, bevorzugt ist das Auskochen in Säuren oder Lösemitteln. So lässt sich durch Auskochen in oxidierenden Säuren eine Carboxylierung dieser aktivierten Kohlenstoffschichten erzeugen.

Vor der medizinischen Verwendung oder einer Beladung mit Wirkstoffen können die erfindungsgemäßen Implantate mit üblichen Methoden sterilisiert werden, beispielsweise durch Autoklavieren, Ethylenoxid-Sterilisation oder Gamma-Bestrahlung.
Erfindungsgemäß lassen sich alle mögliche Aktivierungsverfahren miteinander sowie auch mit beliebigen der nachfolgend beschriebenen Funktionalisierungsschritten kombinieren.

### FUNKTIONALISIERUNG

Die Implantate können durch geeignete Maßnahmen zusätzlich mit einer Vielzahl von Funktionen ausgestattet werden. Orthopädische und chirurgische Implantate oder Gefäßendoprothesen können als Arzneimittelträger oder -depots verwendet werden. Die Biokompatibilität und die Funktionalität der erfindungsgemäßen Implantate kann durch den Einbau von Zusatzstoffen, Füllstoffen, Proteinen gezielt beeinflusst bzw. verändert werden. Hierdurch lassen sich Abstoßungsphänomene im Körper bei erfindungsgemäß hergestellten Implantaten verringern oder ganz ausschalten oder die Wirksamkeit des Implantats steigern bzw. zusätzliche Wirkungen erzeugen.

Unter Funktionalisierung im Sinne der vorliegenden Erfindung werden allgemein Maßnahmen verstanden, in deren Folge die kohlenstoffhaltige Schicht weitere, zusätzliche Funktionen erhält. Erfindungsgemäße Funktionalisierungen bestehen im Einbau von Stoffen in die kohlenstoffhaltige Schicht oder die Fixierung von Stoffen an der kohlenstoffhaltigen Schicht. Geeignete Stoffe werden ausgewählt aus pharmakologischen Wirkstoffen, Linker, Mikroorganismen, pflanzliche oder tierische einschließlich menschlicher Zellen oder Zellkulturen und Gewebe, Mineralien, Salze, Metalle, synthetische oder natürliche Polymere, Proteine, Peptide, Aminosäuren, Lösemittel etc.

Erfindungsgemäß kann das geeignet aktivierte Implantat funktionalisiert werden, indem es vor oder nach einer möglichen Beladung mit Wirkstoffen dadurch bioverträglicher gemacht wird, dass es mit mindestens einer zusätzlichen Schicht aus biologisch abbaubaren bzw. resorbierbaren Polymeren wie Kollagen, Albumin, Gelatine, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvalerinsäure), Polydioxanone, Poly(Ethylenterephtalate), Poly(malatsäure), Poly(Tartronsäure), Polyanhydride, Polyphosphazene, Poly(Aminosäuren), und deren Co-Polymere oder nicht-biologisch abbaubaren bzw. resorbierbaren Polymeren zumindest teilweise zu beschichten. Bevorzugt sind insbesondere anionische, kationischen oder amphotere Beschichtungen, wie z.B. Alginat, Carrageenan, Carboxymethylcellulose; Chitosan, Poly-L-Lysine; und/oder Phoshporylcholin ausgestattet wird.

Auch können im Funktionalisierungsschritt des erfindungsgemäßen Verfahrens auf die aktivierte kohlenstoffhaltige Schicht Wirkstoffe wie Arzneimittel und Medikamente aufgebracht oder in die Schicht eingebracht werden. Dies ist insbesondere da nützlich, wo Wirkstoffe nicht im oder auf dem Implantat direkt aufgebracht werden können, wie etwa bei Metallen.

So lassen sich beispielsweise auf metallischen Oberflächen schwer wasserlösliche, lipophile Wirkstoffe wie Paclitaxel auftragen, welche zur Bildung eines kristallinen Films neigen. Üblicherweise sind die immobilisierbaren Mengen begrenzt und die Freisetzung nicht kontrollierbar. Eine direkte Beschichtung von solchen metallischen Oberflächen mit Paclitaxel führt zu maximalen Beladungen von etwa 3mg/mm², deren Freisetzung unter physiologischen Bedingungen in physiologischen Pufferlösungen zu einer unkontrollierten Desorption von maximal 30% innerhalb von 1 bis 5 Tagen führt.

Erfindungsgemäß aktivierte Kohlenstoffschichten vorzugsweise glasartig amorphe, mit einer Schichtdicke im Bereich von 80 nm bis 10 µm, bevorzugt von 100 nm bis 5 µm, vorzugsweise mit einer Porosität von 5 nm bis 1 µm, bevorzugt von 5 nm bis 1000 nm, können beispielsweise bereits bei Porositäten von 5% bis 50%, bevorzugt von 10% bis 50%, und einer mittleren Porengröße von 5 nm bis 1 µm, bevorzugt von 5 nm bis 500 nm, Wirkstoffmengen bis hin zum hundertfachen dessen von nichtaktivierten kohlenstoffbeschichteten oder rein metallischen Implantaten aufnehmen und in Abhängigkeit der Porosität bzw. der Porengröße und der Oberflächeneigenschaften gegebenenfalls kontrolliert freisetzen.

Bei Beladungsmengen von 0,5 bis 3,0 µg/mm² Paclitaxel und hydrophoben Kohlenstoffoberflächen mit einer Schichtdicke von 200 nm können in einer erfindungsgemäßen Ausführungsform mit 50 nm Porengröße und einem Porositätsgrad von 5% beispielsweise gezielt mit einer konstanten täglichen Freisetzungsrate 70-100% der aufgebrachten Paclitaxelmenge innerhalb von 25 bis 35 Tagen unter physiologischen Bedingungen kontrolliert freigesetzt werden.

In besonders bevorzugten Ausführungsformen können durch geeignete Funktionalisierung der beliebigen kohlenstoffhaltigen Schicht auch Peptide und Proteine sowie Glykoproteine und Lipoproteine immobilisiert werden.

Eine erfindungsgemäße Form der Funktionalisierung besteht in der kovalenten oder nicht-kovalenten Adsorption von Substanzen, welche die Bindung von mit Affinitätsanhängseln (so genannte affinity tags) versehenen bzw. markierten Peptiden, Proteinen, Glykoproteinen oder Lipoproteinen erlauben.

Derartige Substanzen sind beispielsweise Ionen, Kationen, insbesondere Metallkationen wie Kobalt-, Nickel-, Kupfer-, Zink-Kationen, Antikörper, Calmodulin, Chitin, Cellulose, Zucker, Aminosäuren, Gluthathion, Streptavidin, Strep-tactin oder andere Mutanten zur Bindung von Strep-tag oder SBP-tag markierten Substanzen, oder S-Protein um S-tag markierte Substanzen zu binden, sowie dergleichen.

Diese Affinitätsanhängsel werden auf geeignete Weise den zu immobilisierenden Peptiden, Proteinen, Glykoproteinen oder Lipoproteinen entweder am C-terminalen oder N-terminalen Ende der Primärsequenz angefügt, üblicherweise auf dem Weg der rekombinanten gentechnischen Herstellung oder Biotynilierung. Bevorzugt sind hierbei Affinitätsanhängsel, insbesondere Polyarginin-Anhängsel (Arg-tag), welche bevorzugt aus fünf bis sechs Argininsäuren bestehen, Polyhistidin-Anhängsel (Histag), einer beliebig langen Polyhistidine-Sequenz, typischerweise 2 bis 10 Reste, FLAG-Anhängsel (FLAG-tag) mit der Sequenz DYKDDDDK, Strep-Anhängsel (Strep-tag), zum Beispiel die Strep-tag II Sequenz WSHPQFEK, S-Anhängsel (S-tag), welches die Aminosärereste KETAAAKFERQHMDS trägt, das Calmodulinbindende Peptid (calmodulin binding peptide), die Familie der Cellulose binding domains, insbesondere C-terminal, N-terminal oder auch anderer Position in der Primärsequenz des zu immobilisierenden Peptides, Proteins, Glyko- oder Lipoproteins, das SBP-Anhängsel (SBP-tag), mit der Sequenz MDEKTTGWRGGHVVEGLAGELEQLRARLEHHPQGQREP, das Polyhistidin-Anhängsel (Polyhistidine-tag), Chitin-bindende Domänen (chitinbinding domains), Gluthation-S-Transferase-tag (Gluthatione-S-Transferase-tag), Maltose bindendes Protein (Maltose-binding Protein), Bacteriophage T7 und V5 epitope, aber auch jedes anderes Affinitätsanhängsel.

Die Modifikation der auf den funktionalisierten Kohlenstoffoberflächen aufzubringenden Substanzen entspricht den üblichen Systemen, welche in der Aufreinigung und insbesondere chromatographischen Markierung möglich sind.

Die Funktionalisierung der Kohlenstoffoberfläche erfolgt hierbei durch die Adsorption von korrespondierenden Substanzen in und/oder an der kohlenstoffhaltigen Schicht, welche mit den Affinitätsanhängseln eine Bindung eingehen können. Entsprechende korrespondierende Substanzen sind beispielsweise Kationen, welche in die Kohlenstoffschicht eingebracht werden, um die Bindung zum basischen Polyarginin-Anhängsel zu ermöglichen, etwa Kobalt-, Nickel-, Kupfer-, Zink-Kationen, um beispielsweise die Bindung von Polyhistidin-Anhängseln zu ermöglichen.

Die Adsoption des Antikörpers M1 auf den Kohlenstoffoberflächen ermöglicht die Bindung von FLAG-Anhängseln, Streptavidin bzw. Strep-tactin oder andere Mutanten zur Bindung von Strep-tag oder SBP-tag markierten Substanzen, oder die Adsorption des S-Proteins auf der Oberfläche, um S-tag markierte Substanzen zu binden.

In einer weiteren Ausführungsform besteht die Funktionalisierung in der Verwendung von Calmodulin, welches auf der Kohlenstoffoberfläche zu adsorbieren ist. Hiermit lassen sich Calmodulin-Binding-Peptide-markierte Substanzen an die kohlenstoffhaltige Schicht binden.

In weiteren Ausführungsformen erfolgt die Funktionalisierung durch Adsorption von Cellulose so dass mit Cellulose-bindenden Domänen modifizierte Substanzen gebunden werden können, oder aber durch Adsorption von Chitin, um mit Chitinbindenden Domänen versehende Substanzen zu binden.

Analog kann mit Gluthathion zur Bindung von Gluthathion-S-Transferase-tag markierte Substanzen funktionalisiert werden, mit Maltose oder Amylose, um mit Maltose-bindendem Protein markierte Substanzen zu binden.

Der Fachmann wird entsprechend den gentechnisch möglichen Bedingungen, den funktionellen und strukturellen Eigenschaften des Peptids, Proteins, Glyko- oder Lipoproteins ein geeignetes Affinitäts-System wählen.

So können beispielsweise auf porösen Kohlenstoffoberflächen mit einer Porengröße von 100-900 nm, einer Porosität von 30-60% und einer Schichtdicke von 1-5µm aus einer Strep-Tactin-Lösung durch Sprühen oder Tauchen Kohlenstoffschichten funktionalisiert mit 0,1 - 8 µg/mm² adsorbiertem Strep-Tactin gewonnen werden. Die auf diese Weise funktionalisierte Kohlenstoffschicht kann beispielsweise 0,1 - 10 µg/mm² rekombinantes, mit dem Strep-tag markiertes IL-2 aufnehmen.

In einer weiteren Ausführungsform wird die Kohlenstoffschicht mit Kobaltionen dotiert, wobei die poröse Kohlenstoffmatrix einen Kobaltionengehalt von 0,1 bis zu 50% des Feststoffgehalts enthält, bevorzugt bis zu 60% in glasartigen porösen Kohlenstoffschichten. Bei einer Porösität von 50%, Schichtdicken von 50nm bis 1000 nm, können durch die Metallionendotierung in der Matrix 0,1 bis 100 µg Polyarginin-tag markiertes rekombinantes IL-2 adsorbiert werden.

Eine weitere Ausführungsform sieht beispielsweise die Funktionalisierung der Kohlenstoffoberflächen mittels Adsorption von Linker-Substanzen vor, bevorzugt von carboxymethylierten Dextranen, zum Beispiel als Hydrogel, welche die physikalische Bindung von Substanzen, bevorzugt Biomolekülen oder Wirkstoffen ermöglichen und bzw. oder chemische Reaktivität besitzen, so dass mittels kovalenter Bindungen solche Substanzen kovalent gebunden werden können, bevorzugt durch die Bildung von Amino-, Thiol- oder Aldehydbindungen.

Der Fachmann wird in Abhängigkeit des Ligandentyps die geeignete Art des Linkers auswählen.

Für die Herstellung einer Aminobindung lässt sich in bevorzugten Ausführungsformen die Kohlenstoffschicht in folgender Weise funktionalisieren: Adsorption von carboxymethyliertem Dextran, nachfolgende Modifikation durch Inkubation in NHS/EDC zur Umwandlung der Carboxymethylgruppen in N-Hydroxysuccinimidester.

Auf diese Weise können Liganden adsorbiert werden, welche kovalente Amino-Bindungen mit den Estern eingehen. Nicht abreagierte Ester können in einem weiteren Schritt durch beispielsweise Inkubation in 1M Ethanolamin-HydrochloridLösung wieder inaktiviert werden. So ergibt beispielsweise die Adsorption von 1µg carboxymethyliertem Dextran pro mm² einer porösen, kohlenstoffhaltigen Kompositschicht aus glasartigem Kohlenstoff und Russpartikeln eine Funktionalisierung, welche 0,01 bis 5000 µg/mm² Peptide mit einem Molekulargewicht von 60-90 kovalent binden kann.

Ferner können die erfindungsgemäß aktivierten, porösen Schichten im Funktionalisierungsschritt des Verfahrens mit Arzneistoffen bzw. Medikamenten, Mikroorganismen, Zellen und/oder Geweben beladen werden, oder auch mit diagnostischen Hilfsmitteln wie Markern oder Kontrastmitteln zur Lokalisierung von beschichteten Implantaten im Körper versehen werden, beispielsweise auch mit therapeutischen oder diagnostischen Mengen an radioaktiven Strahlern.

### WIRKSTOFFBESCHICHTUNG

In bevorzugten Ausführungsformen werden die erfindungsgemäß aktivierten Implantate im Funktionalisierungsschritt mit Wirkstoffen beladen. Die Beladung mit Wirkstoffen kann in oder auf der kohlenstoffhaltigen Schicht mittels geeigneter sorptiver Methoden wie Adsorption, Absorption, Physisorption, Chemisorption erfolgen, im einfachsten Fall durch Imprägnierung der kohlenstoffhaltigen Beschichtung mit Wirkstofflösungen, Wirkstoffdispersionen oder Wirkstoffsuspensionen in geeigneten Lösungsmitteln. Auch kovalente oder nichtkovalente Anbindung von Wirkstoffen in oder auf der kohlenstoffhaltigen Beschichtung kann hier nach Abhängigkeit des verwendeten Wirkstoffs und seiner chemischen Eigenschaften eine bevorzugte Option sein.

In bevorzugten Ausführungsformen wird der Wirkstoff in Form einer Lösung, Dispersion oder Suspension in einem geeigneten Lösemittel oder Lösemittelgemisch, ggf. mit anschließender Trocknung, aufgetragen. Geeignete Lösemittel umfassen beispielsweise Methanol, Ethanol, N-Propanol, Isopropanol, Butoxydiglycol, Butoxyethanol, Butoxyisopropanol, Butoxypropanol, n-Butyl-Alkohol, t-Butyl-Alkohol, Butyleneglycol, Butyloctanol, Diethylenglycol, Dimethoxydiglycol, Dimethylether, Dipropylenglycol, Ethoxydiglycol, Ethoxyethanol, Ethylhexandiol, Glycol, Hexanediol, 1,2,6-Hexanetriol, Hexylalkohol, Hexylenglycol, Isobutoxypropanol, Isopentyldiol, 3-Methoxybutanol, Methoxydiglycol, Methoxyethanol, Methoxyisopropanol, Methoxymethylbutanol, Methoxy PEG-10, Methylal, Methyl-Hexylether, Methylpropanediol, Neopentylglycol, PEG-4, PEG-6, PEG-7, PEG-8, PEG-9, PEG-6-Methylether, Pentylenglycol, PPG-7, PPG-2-Buteth-3, PPG-2 Butylether, PPG-3 Butylether, PPG-2 Methylether, PPG-3 Methylether, PPG-2 Propylether, Propanediol, Propylenglycol, Propylenglycol-Butylether, Propylenglycol-Propylether, Tetrahydrofuran, Trimethylhexanol, Phenol, Benzol, Toluol, Xylol; als auch Wasser, ggf. im Gemisch mit Dispersionshilfsmitteln, sowie Mischungen der obengenannten davon.

Bevorzugte Lösungsmittel umfassen ein oder mehrere organische Lösungsmittel aus der Gruppe Ethanol, Isopropanol, *n*-Propanol, Dipropylenglykolmethylether und Butoxyisopropanol (1,2-Propylenglykol-*n*-butylether), Tetrahydrofuran, Phenol, Benzol, Toluol, Xylol, vorzugsweise Ethanol, Isopropanol, *n*-Propanol und/oder Dipropylenglykolmethylether, insbesondere Isopropanol und/oder *n*-Propanol.

In den aktivierten, porösen kohlenstoffhaltigen Schichtungen können geeignet dimensionierte Wirkstoffe auch in Poren okkludiert werden.

Die Wirkstoffbeladung kann temporär sein, d. h. der Wirkstoff kann nach Implantierung der medizinischen Vorrichtung freigesetzt werden, oder aber der Wirkstoff wird in oder auf der kohlenstoffhaltigen Schicht dauerhaft immobilisiert. Auf diese Weise können wirkstoffhaltige medizinische Implantate mit statischen, dynamischen oder kombiniert statischen und dynamischen Wirkstoffbeladungen erzeugt werden. So ergeben sich multifunktionale Beschichtungen auf Basis der erfindungsgemäß hergestellten kohlenstoffhaltigen Schichten.

Bei statischer Beladung mit Wirkstoffen werden Wirkstoffe im Wesentlichen permanent auf oder in der Beschichtung immobilisiert. Hierfür verwendbare Wirkstoffe sind anorganische Substanzen, z.B. Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, sowie pharmakologisch wirksame Stoffe oder Stoffgemische, Kombinationen dieser und dergleichen.

Bei dynamischen Wirkstoffbeladungen ist die Freisetzung der aufgebrachten Wirkstoffe nach Implantierung der medizinischen Vorrichtung im Körper vorgesehen. Auf diese Weise können die beschichteten Implantate zu therapeutischen Zwecken eingesetzt werden, wobei die auf das Implantat aufgebrachten Wirkstoffe lokal am Einsatzort des Implantats sukzessive freigesetzt werden. In dynamischen Wirkstoffbeladungen für die Freisetzung von Wirkstoffen verwendbare Wirkstoffe sind beispielsweise Hydroxylapatit (HAP), Fluorapatit, Trikalziumphosphat (TCP), Zink; und/oder organische Substanzen wie Peptide, Proteine, Kohlenhydrate wie Mono-, Oligo- und Polysaccharide, Lipide, Phospholipide, Steroide, Lipoproteine, Glykoproteine, Glykolipide, Proteoglykane, DNA, RNA, Signalpeptide oder Antikörper bzw. Antikörperfragmente, bioresorbierbare Polymere, z.B. Polylactonsäure, Chitosan, und dergleichen, sowie pharmakologisch wirksame Stoffe oder Stoffgemische.

Geeignete pharmakologisch wirksame Stoffe oder Stoffgemische zur statischen und/oder dynamischen Beladung von erfindungsgemäß beschichteten implantierbaren medizinischen Vorrichtungen umfassen Wirkstoffe oder Wirkstoffkombinationen, die ausgewählt sind aus Heparin, synthetische Heparin-Analoga (z.B. Fondaparinux), Hirudin, Antithrombin III, Drotrecogin alpha; Fibrinolytica wie Alteplase, Plasmin, Lysokinasen, Faktor XIIa, Prourokinase, Urokinase, Anistreplase, Streptokinase; Thrombozytenaggregations-hemmer wie Acetylsalicylsäure, Ticlopidine, Clopidogrel, Abciximab, Dextrane; Corticosteroide wie Alclometasone, Amcinonide, Augmented Betamethasone, Beclomethasone, Betamethasone, Budesonide, Cortisone, Clobetasol, Clocortolone, Desonide, Desoximetasone, Dexamethasone, Flucinolone, Fluocinonide, Flurandrenolide, Flunisolide, Fluticasone, Halcinonide, Halobetasol, Hydrocortisone, Methylprednisolone, Mometasone, Prednicarbate, Prednisone, Prednisolone, Triamcinolone; sogenannte Non-Steroidal Anti-Inflammatory Drugs wie Diclofenac, Diflunisal, Etodolac, Fenoprofen, Flurbiprofen, Ibuprofen, Indomethacin, Ketoprofen, Ketorolac, Meclofenamate, Mefenamic acid, Meloxicam, Nabumetone, Naproxen, Oxaprozin, Piroxicam, Salsalate, Sulindac, Tolmetin, Celecoxib, Rofecoxib; Zytostatika wie Alkaloide und Podophyllumtoxine wie Vinblastin, Vincristin; Alkylantien wie Nitrosoharnstoffe, Stickstofflost-Analoga; zytotoxische Antibiotika wie Daunorubicin, Doxorubicin und andere Anthrazykline und verwandte Substanzen, Bleomycin, Mitomycin; Antimetabolite wie Folsäure-, Purinoder Pyrimidin-Analoga; Paclitaxel, Docetaxel, Sirolimus; Platinverbindungen wie Carboplatin, Cisplatin oder Oxaliplatin; Amsacrin, Irinotecan, Imatinib, Topotecan, Interferon-alpha 2a, Interferon-alpha 2b, Hydroxycarbamid, Miltefosin, Pentostatin, Porfimer, Aldesleukin, Bexaroten, Tretinoin; Antiandrogene, und Antiöstrogene; Antiarrythmika, insbesondere Antiarrhythmika der Klasse I wie Antiarrhythmika vom Chinidintyp, z.B. Chinidin, Dysopyramid, Ajmalin, Prajmaliumbitartrat, Detajmiumbitartrat; Antiarrhythmika vom Lidocaintyp, z.B. Lidocain, Mexiletin, Phenytoin, Tocainid; Antiarrhythmika der Klasse I C, z.B. Propafenon, Flecainid(acetat); Antiarrhythmika der Klasse II, Betarezeptorenblocker wie Metoprolol, Esmolol, Propranolol, Metoprolol, Atenolol, Oxprenolol; Antiarrhythmika der Klasse III wie Amiodaron, Sotalol; Antiarrhythmika der Klasse IV wie Diltiazem, Verapamil, Gallopamil; andere Antiarrhythmika wie Adenosin, Orciprenalin, Ipratropiumbromid; Agenzien zur Stimulation der Angiogenese im Myokard wie Vascular Endothelial Growth Factor (VEGF), Basic Fibroblast Growth Factor (bFGF), nicht virale DNA, virale DNA, endotheliale Wachstumsfaktoren: FGF-1, FGF-2, VEGF, TGF; Antikörper, Monoklonale Antikörper, Anticaline; Stammzellen, Endothelial Progenitor Cells (EPC); Digitalisglykoside wie Acetyldigoxin/Metildigoxin, Digitoxin, Digoxin; Herzglykoside wie Ouabain, Proscillaridin; Antihypertonika wie zentral wirksame antiadrenerge Substanzen, z.B. Methyldopa, Imidazolinrezeptoragonisten; Kalciumkanalblocker vom Dihydropyridintyp wie Nifedipin, Nitrendipin; ACE-Hemmer: Quinaprilat, Cilazapril, Moexipril, Trandolapril, Spirapril, Imidapril, Trandolapril; Angiotensin-II-Antagonisten: Candesartancilexetil, Valsartan, Telmisartan, Olmesartanmedoxomil, Eprosartan; peripher wirksame alpha-Rezeptorenblocker wie Prazosin, Urapidil, Doxazosin, Bunazosin, Terazosin, Indoramin; Vasodilatatoren wie Dihydralazin, Diisopropylamindichloracetat, Minoxidil, Nitroprussidnatrium; andere Antihypertonika wie Indapamid, Co-Dergocrinmesilat, Dihydroergotoxinmethansulfonat, Cicletanin, Bosentan, Fludrocortison; Phosphodiesterasehemmer wie Milrinon, Enoximon und Antihypotonika, wie insbesondere adrenerge und dopaminerge Substanzen wie Dobutamin, Epinephrin, Etilefrin, Norfenefrin, Norepinephrin, Oxilofrin, Dopamin, Midodrin, Pholedrin, Ameziniummetil; und partielle Adrenozeptor-Agonisten wie Dihydroergotamin; Fibronectin, Polylysine, Ethylenevinylacetate, inflammatorische Zytokine wie: TGFβ, PDGF, VEGF, bFGF, TNFα, NGF, GM-CSF, IGF-α, IL-1, IL-8, IL-6, Growth Hormone; sowie adhäsive Substanzen wie Cyanacrylate, Beryllium, Silica; und Wachstumsfaktoren (Growth Factor) wie Erythropoetin, Hormonen wie Corticotropine, Gonadotropine, Somatropin, Thyrotrophin, Desmopressin, Terlipressin, Oxytocin, Cetrorelix, Corticorelin, Leuprorelin, Triptorelin, Gonadorelin, Ganirelix, Buserelin, Nafarelin, Goserelin, sowie regulatorische Peptide wie Somatostatin, Octreotid; Bone and Cartilage Stimulating Peptides, bone morphogenetic proteins (BMPs), insbesondere rekombinante BMP's wie z.B. Recombinant human BMP-2 (rhBMP-2)), Bisphosphonate (z.B. Risedronate, Pamidronate, Ibandronate, Zoledronsäure, Clodronsäure, Etidronsäure, Alendronsäure, Tiludronsäure), Fluoride wie Dinatriumfluorophosphat, Natriumfluorid; Calcitonin, Dihydrotachystyrol; Growth Factors und Zytokine wie Epidermal Growth Factor (EGF), Platelet-Derived Growth Factor (PDGF), Fibroblast Growth Factors (FGFs), Transforming Growth Factors-b TGFs-b), Transforming Growth Factor-a (TGF-a), Erythropoietin (Epo), Insulin-Like Growth Factor-I (IGF-I), Insulin-Like Growth Factor-II (IGF-II), Interleukin-1 (IL-1), Interleukin-2 (IL-2), Interleukin-6 (IL-6), Interleukin-8 (IL-8), Tumor Necrosis Factor-a (TNF-a), Tumor Necrosis Factor-b (TNF-b), Interferon-g (INF-g), Colony Stimulating Factors (CSFs); Monocyte chemotactic protein, fibroblast stimulating factor 1, Histamin, Fibrin oder Fibrinogen, Endothelin-1, Angiotensin II, Kollagene, Bromocriptin, Methylsergid, Methotrexat, Kohlenstofftetrachlorid, Thioacetamid, und Ethanol; ferner Silber(ionen), Titandioxid, Antibiotika und Antiinfektiva wie insbesondere β -Laktam-Antibiotika, z.B. β-Lactamase-sensitive Penicilline wie Benzylpenicilline (Penicillin G), Phenoxymethylpenicillin (Penicillin V); β-Lactamase-resistente Penicilline wie Aminopenicilline wie Amoxicillin, Ampicillin, Bacampicillin; Acylaminopenicilline wie Mezlocillin, Piperacillin; Carboxypenicilline, Cephalosporine wie Cefazolin, Cefuroxim, Cefoxitin, Cefotiam, Cefaclor, Cefadroxil, Cefalexin, Loracarbef, Cefixim, Cefuroximaxetil, Ceftibuten, Cefpodoximproxetil, Cefpodoximproxetil; Aztreonam, Ertapenem, Meropenem; β-Lactamase-Inhibitoren wie Sulbactam, Sultamicillintosilat; Tetracycline wie Doxycyclin, Minocyclin, Tetracyclin, Chlortetracyclin, Oxytetracyclin; Aminoglykoside wie Gentamicin, Neomycin, Streptomycin, Tobramycin, Amikacin, Netilmicin, Paromomycin, Framycetin, Spectinomycin; Makrolidantibiotika wie Azithromycin, Clarithromycin, Erythromycin, Roxithromycin, Spiramycin, Josamycin; Lincosamide wie Clindamycin, Lincomycin, Gyrasehemmer wie Fluorochinolone wie Ciprofloxacin, Ofloxacin, Moxifloxacin, Norfloxacin, Gatifloxacin, Enoxacin, Fleroxacin, Levofloxacin; Chinolone wie Pipemidsäure; Sulfonamide, Trimethoprim, Sulfadiazin, Sulfalen; Glykopeptidantibiotika wieVancomycin, Teicoplanin; Polypeptidantibiotika wie Polymyxine wie Colistin, Polymyxin-B, Nitroimidazol-Derivate wie Metronidazol, Tinidazol; Aminochinolone wie Chloroquin, Mefloquin, Hydroxychloroquin; Biguanide wie Proguanil; Chininalkaloide und Diaminopyrimidine wie Pyrimethamin; Amphenicole wie Chloramphenicol; Rifabutin, Dapson, Fusidinsäure, Fosfomycin, Nifuratel, Telithromycin, Fusafungin, Fosfomycin, Pentamidindiisethionat, Rifampicin, Taurolidin, Atovaquon, Linezolid; Virustatika wie Aciclovir, Ganciclovir, Famciclovir, Foscarnet, Inosin-(Dimepranol-4-acetamidobenzoat), Valganciclovir, Valaciclovir, Cidofovir, Brivudin; antiretrovirale Wirkstoffe (nukleosidanaloge Reverse-Transkriptase-Hemmer und -Derivate) wie Lamivudin, Zalcitabin, Didanosin, Zidovudin, Tenofovir, Stavudin, Abacavir; nicht nukleosidanaloge Reverse-Transkriptase-Hemmer: Amprenavir, Indinavir, Saquinavir, Lopinavir, Ritonavir, Nelfinavir; Amantadin, Ribavirin, Zanamivir, Oseltamivir und Lamivudin, sowie beliebige Kombinationen und Gemische davon.

### STENTS

Besonders bevorzugte Ausführungsformen der vorliegenden Erfindung sind beschichtete Gefäßendoprothesen (intraluminale Endoprothesen) wie Stents, Koronarstents, intravaskulare Stents, periphere Stents und dergleichen.

Diese können mit dem erfindungsgemäßen Verfahren auf einfache Weise biokompatibel funktionalisiert werden, wodurch beispielsweise die in der perkutanen transluminalen Angioplastie mit herkömmlichen Stents häufig auftretenden Restenosen verhindert werden können.

So kann durch die Immobilisierung von geeigneten Wirkstoffen auf porösen kohlenstoffhaltigen Beschichtungen, insbesondere Paclitaxel, Rapamycine oder Dexamethason, die lokale Entzündungsreaktion im Gewebe der Gefäßwand durch vorübergehende lokale Freisetzung dieser Wirkstoffe gehemmt bzw. unterdrückt werden. Die Verwendung und Wirksamkeit solcher Wirkstoffe ist dem Stand der Technik gemäß hinreichend bekannt. Jedoch ist Anwendbarkeit durch die dem Stand der Technik entsprechenden Beschichtungssysteme limitiert, insbesondere wegen der unzureichenden Beladungsfähigkeit, welche zu unzureichender Bioverfügbarkeit führt, unzureichende bzw. unvollständige Freisetzung dieser Wirkstoffe oder Unverträglichkeiten zwischen Beschichtungssystem und Wirkstoff durch unerwünschte physikalische oder chemische Wechselwirkungen.

In bevorzugten Ausführungsformen der vorliegenden Erfindung werden glasartige Kohlenstoffschichten oder Komposit-Schichten mit Russpartikelzusatz mit Schichtdicken zwischen 80 nm und 10 µm, Porengrößen von 5 nm bis 1 µm und Porositäten von 1 % bis 70% erzeugt und aktiviert, bevorzugt durch Einbringen von Füllstoffen und deren anschließender Entfernung aus der Kohlenstoffschicht oder durch die, eine poröse Matrix erzeugende Beimengung von Russpartikeln mit sphärischer oder ellipsoider oder stäbchenförmigen Morphologie und einer Partikelgröße von 10 nm bis 200 nm, so dass Wirkstoffe in ausreichender Menge aufgenommen werden können. Die Oberfläche des Stent-Implantats kann dabei auf bis zu 2000m²/m³ erhöht werden.

In bevorzugten Ausführungen der Erfindung kann durch Aktivierung der kohlenstoffhaltigen Schicht, beispielsweise mit Luft bei erhöhter Temperatur, die Hydrophilie der Beschichtung erhöht werden, was einerseits die Bioverträglichkeit zusätzlich steigert, andererseits die Schicht aufnahmefähiger für Wirkstoffe, insbesondere hydrophile Wirkstoffe macht.

In besonders bevorzugten Ausführungsformen werden nach dem erfindungsgemäßen Verfahren Stents, insbesondere Koronarstents und periphere Stents mit pharmakologisch wirksamen Stoffen oder Stoffgemischen beladen oder mit Zellen oder mit Zellkulturen. Beispielsweise können die kohlenstoffhaltigen Stentoberflächen für die lokale Unterdrückung von Zelladhäsion, Thrombozytenaggregation, Komplementaktivierung bzw. inflammatorische Gewebereaktionen oder Zellproliferation mit folgenden Wirkstoffen ausgerüstet werden mit:

Heparin, synthetische Heparin-Analoga (z.B. Fondaparinux), Hirudin, Antithrombin III, Drotrecogin alpha, Fibrinolytica (Alteplase, Plasmin, Lysokinasen, Faktor XIIa, Prourokinase, Urokinase, Anistreplase, Streptokinase), Thrombozytenaggregations-hemmer (Acetylsalicylsäure, Ticlopidine, Clopidogrel, abciximab, Dextrane), Corticosteroide (Alclometasone, Amcinonide, Augmented Betamethasone, Beclomethasone, Betamethasone, Budesonide, Cortisone, Clobetasol, Clocortolone, Desonide, Desoximetasone, Dexamethasone, Flucinolone, Fluocinonide, Flurandrenolide, Flunisolide, Fluticasone, Halcinonide, Halobetasol, Hydrocortisone, Methylprednisolone, Mometasone, Prednicarbate, Prednisone, Prednisolone, Triamcinolone), sogenannte Non-Steroidal Anti-Inflammatory Drugs (Diclofenac, Diflunisal, Etodolac, Fenoprofen, Flurbiprofen, Ibuprofen, Indomethacin, Ketoprofen, Ketorolac, Meclofenamate, Mefenamic acid, Meloxicam, Nabumetone, Naproxen, Oxaprozin, Piroxicam, Salsalate, Sulindac, Tolmetin, Celecoxib, Rofecoxib), Zytostatika (Alkaloide und Podophyllumtoxine wie Vinblastin, Vincristin; Alkylantien wie Nitrosoharnstoffe, Stickstofflost-Analoga; zytotoxische Antibiotika wie Daunorubicin, Doxorubicin und andere Anthrazykline und verwandte Substanzen, Bleomycin, Mitomycin; Antimetabolite wie Folsäure-, Purinoder Pyrimidin-Analoga; Paclitaxel, Docetaxel, Sirolimus; Platinverbindungen wie Carboplatin, Cisplatin oder Oxaliplatin; Amsacrin, Irinotecan, Imatinib, Topotecan, Interferon-alpha 2a, Interferon-alpha 2b, Hydroxycarbamid, Miltefosin, Pentostatin, Porfimer, Aldesleukin, Bexaroten, Tretinoin; Antiandrogene, Antiöstrogene).

Für systemische, kardiologische Wirkungen können die erfindungsgemäß aktivierten Stents beladen werden mit:

Antiarrythmika, insbesondere Antiarrhythmika der Klasse I (Antiarrhythmika vom Chinidintyp: Chinidin, Dysopyramid, Ajmalin, Prajmaliumbitartrat, Detajmiumbitartrat; Antiarrhythmika vom Lidocaintyp: Lidocain, Mexiletin, Phenytoin, Tocainid; Antiarrhythmika der Klasse I C: Propafenon, Flecainid(acetat)), Antiarrhythmika der Klasse II (Betarezeptorenblocker) (Metoprolol, Esmolol, Propranolol, Metoprolol, Atenolol, Oxprenolol), Antiarrhythmika der Klasse III (Amiodaron, Sotalol), Antiarrhythmika der Klasse IV (Diltiazem, Verapamil, Gallopamil), andere Antiarrhythmika wie Adenosin, Orciprenalin, Ipratropiumbromid; Stimulation der Angiogenese im Myokard: Vascular Endothelial Growth Factor (VEGF), Basic Fibroblast Growth Factor (bFGF), nicht virale DNA, virale DNA, endotheliale Wachstumsfaktoren: FGF-1, FGF-2, VEGF, TGF; Antikörper, Monoklonale Antikörper, Anticaline; Stammzellen, Endothelial Progenitor Cells (EPC). Weitere Kardiaka sind: Digitalisglykoside (Acetyldigoxin/Metildigoxin, Digitoxin, Digoxin), weitere Herzglykoside (Ouabain, Proscillaridin). Ferner Antihypertonika (zentral wirksame antiadrenerge Substanzen: Methyldopa, Imidazolinrezeptoragonisten; Kalciumkanalblocker: vom Dihydropyridintyp wie Nifedipin, Nitrendipin; ACE-Hemmer: Quinaprilat, Cilazapril, Moexipril, Trandolapril, Spirapril, Imidapril, Trandolapril; Angiotensin-II-Antagonisten: Candesartancilexetil, Valsartan, Telmisartan, Olmesartanmedoxomil, Eprosartan; peripher wirksame alpha-Rezeptorenblocker: Prazosin, Urapidil, Doxazosin, Bunazosin, Terazosin, Indoramin; Vasodilatatoren: Dihydralazin, Diisopropylamindichloracetat, Minoxidil, Nitroprussidnatrium), andere Antihypertonika wie Indapamid, Co-Dergocrinmesilat, Dihydroergotoxinmethansulfonat, Cicletanin, Bosentan. Weiters Phosphodiesterasehemmer (Milrinon, Enoximon) und Antihypotonika, hier insbesondere adrenerge und dopaminerge Substanzen (Dobutamin, Epinephrin, Etilefrin, Norfenefrin, Norepinephrin, Oxilofrin, Dopamin, Midodrin, Pholedrin, Ameziniummetil), partielle Adrenozeptor-Agonisten (Dihydroergotamin), schließlich andere Antihypotonika wie Fludrocortison.

Für die Steigerung der Gewebeadhäsion, insbesondere bei peripheren Stents können Komponenten der extrazellulären Matrix, Fibronectin, Polylysine, Ethylenevinylacetate, inflammatorische Zytokine wie: TGFβ, PDGF, VEGF, bFGF, TNFα, NGF, GM-CSF, IGF-α, IL-1, IL-8, IL-6, Growth Hormone; sowie adhäsive Substanzen wie: Cyanoacrylate, Beryllium, oder Silica verwendet werden.

Weitere hierfür geeignete Substanzen, systemisch und/oder lokal wirkend, sind Wachstumsfaktoren (Growth Factor), Erythropoetin.

Auch Hormone können in den Stentbeschichtungen vorgesehen werden, wie beispielsweise Corticotropine, Gonadotropine, Somatropin, Thyrotrophin, Desmopressin, Terlipressin, Oxytocin, Cetrorelix, Corticorelin, Leuprorelin, Triptorelin, Gonadorelin, Ganirelix, Buserelin, Nafarelin, Goserelin, sowie regulatorische Peptide wie Somatostatin, und/oder Octreotid.

Weitere Ausführungsformen sehen eine Funktionalisierung durch Beladung der Kohlenstoffoberflächen mit Zellen vor, beispielsweise mit pluripotenten Stammzellen, Endothelzellen oder Bindegewebszellen. Diese können aus Organismen gewonnen werden, im Labor aus Zellkulturen kultiviert werden oder gentechnisch verändert sein.

So können beispielsweise in einer besonderen Ausführungsform mit aktivierten Kohlenstoffschichten versehene Gefäßimplantate mit Endothelzellkulturen beladen werden, indem diese zuvor in einem Bioreaktor als Substrat bzw. als Aufzucht und Trägersystem für Zellkulturen verwendet werden. Geeignete Verfahren hierzu sind in DE 103 35 131 bzw. PCT/EP04/00077 beschrieben, deren Offenbarung hiermit einbezogen wird.

So können beispielsweise erfindungsgemäße, nanoporös aktivierte Kohlenstoffschichten mit einer Oberfläche von 200 bis 3000 m²/m³ nach Kultivierung mit Endothelzellen beladen werden, wobei die möglichen Zelldichten von 10¹ - 10¹⁶ Zellen/ml Schichtvolumen, bevorzugt von 10³-10¹² Zellen/ml reichen.

### ORTHOPÄDISCHE IMPLANTATE

Im Falle von chirurgischen und orthopädischen Implantaten kann es vorteilhaft sein, die Implantate mit einer oder mehreren kohlenstoffhaltigen Schichten so zu aktivieren, dass die Schichten makroporös sind. Geeignete Porengrößen liegen im Bereich von 0,1 bis 1000 µm, bevorzugt bei 1 bis 400 µm, um eine bessere Integration der Implantate durch Einwachsen ins umliegende Zell- oder Knochengewebe zu unterstützen.

Für orthopädische und nichtorthopädische Implantate sowie erfindungsgemäß funktionalisierte Herzklappen oder Kunstherzteile können ferner, sofern diese mit Wirkstoffen beladen werden sollen, für die lokale Unterdrückung von Zelladhäsion, Thrombozytenaggregation, Komplementaktivierung bzw. inflammatorische Gewebereaktion oder Zellproliferation die gleichen Wirkstoffe eingesetzt werden wie in den oben beschriebenen Stentanwendungen.

Ferner können zur Stimulation von Gewebewachstum insbesondere bei orthopädischen Implantaten für eine bessere Implantatintegration folgende Wirkstoffe verwendet werden: Bone and Cartilage Stimulating Peptides, bone morphogenetic proteins (BMPs), insbesondere rekombinante BMP's (z.B. Recombinant human BMP-2 (rhBMP-2)), Bisphosphonate (z.B. Risedronate, Pamidronate, Ibandronate, Zoledronsäure, Clodronsäure, Etidronsäure, Alendronsäure, Tiludronsäure), Fluoride (Dinatriumfluorophosphat, Natriumfluorid); Calcitonin, Dihydrotachystyrol. Dann alle Growth Factors und Zytokine (Epidermal Growth Factor (EGF), Platelet-Derived Growth Factor (PDGF), Fibroblast Growth Factors (FGFs), Transforming Growth Factors-b TGFs-b), Transforming Growth Factor-a (TGF-a), Erythropoietin (Epo), Insulin-Like Growth Factor-I (IGF-I), Insulin-Like Growth Factor-II (IGF-II), Interleukin-1 (IL-1), Interleukin-2 (IL-2), Interleukin-6 (IL-6), Interleukin-8 (IL-8), Tumor Necrosis Factor-a (TNF-a), Tumor Necrosis Factor-b (TNF-b), Interferon-g (INF-g), Colony Stimulating Factors (CSFs)). Weitere adhäsions- und integrationsfördernde Substanzen sind neben den bereits genannten inflammatorischen Cytokinen das Monocyte chemotactic protein, fibroblast stimulating factor 1, Histamin, Fibrin oder Fibrinogen, Endothelin-1, Angiotensin II, Kollagene, Bromocriptin, Methylsergid, Methotrexat, Kohlenstofftetrachlorid, Thioacetamid, Ethanol.

### BESONDERE AUSFÜHRUNGSFORMEN

Darüber hinaus können die erfindungsgemäß aktivierten Implantate, Stents und dergleichen auch anstelle von Pharmazeutika oder zusätzlich mit antibakteriellenantiinfektiösen Beschichtungen oder Imprägnierungen versehen werden, wobei die folgenden Stoffe oder Stoffgemische verwendbar sind: Silber(ionen), Titandioxid, Antibiotika und Antiinfektiva. Insbesondere beta-Laktam-Antibiotika (β-Lactam-Antibiotika: β-Lactamase-sensitive Penicilline wie Benzylpenicilline (Penicillin G), Phenoxymethylpenicillin (Penicillin V); β-Lactamase-resistente Penicilline wie Aminopenicilline wie Amoxicillin, Ampicillin, Bacampicillin; Acylaminopenicilline wie Mezlocillin, Piperacillin; Carboxypenicilline, Cephalosporine (Cefazolin, Cefuroxim, Cefoxitin, Cefotiam, Cefaclor, Cefadroxil, Cefalexin, Loracarbef, Cefixim, Cefuroximaxetil, Ceftibuten, Cefpodoximproxetil, Cefpodoximproxetil) oder andere wie Aztreonam, Ertapenem, Meropenem. Weitere Antibiotika sind β-Lactamase-Inhibitoren (Sulbactam, Sultamicillintosilat), Tetracycline (Doxycyclin, Minocyclin, Tetracyclin, Chlortetracyclin, Oxytetracyclin), Aminoglykoside (Gentamicin, Neomycin, Streptomycin, Tobramycin, Amikacin, Netilmicin, Paromomycin, Framycetin, Spectinomycin), Makrolidantibiotika (Azithromycin, Clarithromycin, Erythromycin, Roxithromycin, Spiramycin, Josamycin), Lincosamide (Clindamycin, Lincomycin), Gyrasehemmer (Fluorochinolone wie Ciprofloxacin, Ofloxacin, Moxifloxacin, Norfloxacin, Gatifloxacin, Enoxacin, Fleroxacin, Levofloxacin; andere Chinolone wie Pipemidsäure), Sulfonamide und Trimethoprim (Sulfadiazin, Sulfalen, Trimethoprim), Glykopeptidantibiotika (Vancomycin, Teicoplanin), Polypeptidantibiotika ( Polymyxine wie Colistin, Polymyxin-B), Nitroimidazol-Derivate ( Metronidazol, Tinidazol), Aminochinolone (Chloroquin, Mefloquin, Hydroxychloroquin), Biguanide (Proguanil), Chininalkaloide und Diaminopyrimidine (Pyrimethamin), Amphenicole (Chloramphenicol) und andere Antibiotika (Rifabutin, Dapson, Fusidinsäure, Fosfomycin, Nifuratel, Telithromycin, Fusafungin, Fosfomycin, Pentamidindiisethionat, Rifampicin, Taurolidin, Atovaquon, Linezolid). Unter den Virustatika sind zu nennen Aciclovir, Ganciclovir, Famciclovir, Foscarnet, Inosin-(Dimepranol-4-acetamidobenzoat), Valganciclovir, Valaciclovir, Cidofovir, Brivudin. Dazu zählen auch Antiretrovirale Wirkstoffe (nukleosidanaloge Reverse-Transkriptase-Hemmer und -Derivate: Lamivudin, Zalcitabin, Didanosin, Zidovudin, Tenofovir, Stavudin, Abacavir; nicht nukleosidanaloge Reverse-Transkriptase-Hemmer: Amprenavir, Indinavir, Saquinavir, Lopinavir, Ritonavir, Nelfinavir) und andere Virustatika wie Amantadin, Ribavirin, Zanamivir, Oseltamivir, Lamivudin.

In besonders bevorzugten Ausführungsformen der vorliegenden Erfindung können die erfindungsgemäßen Implantate mit kohlenstoffhaltigen Schichten vor oder nach der Wirkstoffbeladung mittels weiterer Agenzien in ihren chemischen oder physikalischen Eigenschaften geeignet modifiziert werden, beispielsweise um die Hydrophilie, Hydrophobie, elektrische Leitfähigkeit, Haftung oder sonstige Oberflächeneigenschaften zu modifizieren. Hierfür einsetzbare Stoffe sind biodegradierbare oder nicht-degradierbare Polymere, wie beispielsweise bei den biodegradierbaren: Kollagene, Albumin, Gelatin, Hyaluronsäure, Stärke, Cellulose (Methylcellulose, Hydroxypropylcellulose, Hydroxypropylmethylcellulose, Carboxymethylcellulose-Phtalat; weiterhin Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvaleric Acid), Polydioxanone, Poly(Ethylen-Terephtalate), Poly(malatsäure), Poly(Tartronsäure), Polyanhydride, Polyphosphohazene, Poly(Aminosäuren), und alle ihre Co-Polymere.

Zu den nicht-biodegradierbaren zählen: Poly(Ethylen-Vinylacetate), Silicone, Acrylpolymere wie Polyacrylsäure, Polymethylacrylsäure, Polyacrylcynoacrylat; Polyethylene, Polypropylene, Polyamide, Polyurethane, Poly(Ester-Urethane), Poly(Ether-Urethane), Poly(Ester-Harnstoffe), Polyether wie Polyethylenoxid, Polypropylenoxid, Pluronics, PolyTetramethylenglycol; Vinylpolymere wie Polyvinylpyrrolidone, Poly(Vinyl-alkohole), Poly(vinyl-acetat-phatalat; Parylene.

Generell gilt, dass Polymere mit anionischen (z.B. Alginat, Carrageenan, carboxymethylcellulose) oder kationischen (z.B. Chitosan, Poly-L-Lysine etc.) oder beiden Eigenschaften (Phoshporylcholin) hergestellt werden können.

Diese Polymere können auf die Oberfläche der Implantate aufgebracht werden, und diese ganz oder teilweise bedecken.

Zur Modifizierung der Freisetzungseigenschaften wirkstoffhaltiger erfindungsgemäßer Implantate können durch Auftragen beispielsweise von weiteren Polymeren spezifische pH- oder temperaturabhängige Freisetzungseigenschaften erzeugt werden. PH-sensitive Polymere sind beispielsweise Poly(Acrylsäure) und Derivate, zum Beispiel: Homopolymere wie Poly(Aminocarboxylsäure), Poly(Acrylsäure), Poly(Methyl-Acrylsäure) und deren Co-Polymere. Ebenso gilt dies für Polysaccharide wie Celluloseacetat-Phtalat, Hydroxypropylmethylcellulose-Phtalat, Hydroxypropylmethylcellulose-Succinat, Celluloseacetat-Trimellitat und Chitosan. Thermosensitive Polymere sind beispielsweise Poly(N-Isopropylacrylamid-Co-Natrium-Acrylat-Co-n-N-Alkylacrylamid), Poly(N-Methyl-N-n-propylacrylamid), Poly(N-Methyl-N-Isopropylacrylamid), Poly(N-n-Propylmethacrylamid), Poly(N-Isopropylacrylamid), Poly(N,n-Diethylacrylamid), Poly(N-Isopropylmethacrylamid), Poly(N-Cyclopropylacrylamid), Poly(N-Ethylacrylamid), Poly(N-Ethylmethyacrylamid), Poly(N-Methyl-N-Ethylacrylamid), Poly(N-Cyclopropylacrylamid). Weitere Polymere mit Thermogel-Charakteristik sind Hydroxypropyl-Cellulose, Methyl-Cellulose, Hydroxypropylmethyl-Cellulose, Ethylhydroxyethyl-Cellulose und Pluronics wie F-127, L-122, L-92, L-81, L-61.

Die Wirkstoffe können einerseits in den Poren der kohlenstoffhaltigen Schicht adsorbiert werden (nicht-kovalent, kovalent), wobei deren Freisetzung primär durch Porengröße und -geometrie steuerbar sind. Zusätzliche Modifikationen der porösen Kohlenstoffschicht durch chemische Modifikation (anionisch, kationisch) erlauben die Freisetzung zu modifizieren, beispielsweise pH-abhängig. Eine weitere Anwendung stellt die Freisetzung von wirkstoffhaltigen Trägern dar, nämlich Microcapsules, Liposomen, Nanocapsules, Nanopartikeln, Micellen, synthetische Phospholipide, Gas-Dispersionen, Emulsionen, Mikroemulsionen, Nanospheres etc., die in den Poren der Kohlenstoffschicht adsorbiert und dann therapeutisch freigesetzt werden. Durch zusätzliche kovalente oder nicht-kovalente Modifikation der Kohlenstoffschicht lassen sich die Poren okkludieren, so dass biologisch aktive Wirkstoffe geschützt sind. In Frage kommen die oben bereits genannten Polysaccharide, Lipide etc., allerdings auch die genannte Polymere.

Bei der zusätzlichen Beschichtung der erfindungsgemäß erzeugten porösen kohlenstoffhaltigen Schichten mit weiteren Schichten kann daher zwischen physischen Barrieren wie inerten biodegradierbaren Substanzen (Poly-1-Lysin, Fibronectin, Chitosan, Heparin etc.) und biologisch aktiven Barrieren unterschieden werden. Letztere können sterisch behindernde Moleküle sein, die physiologisch bioaktiviert werden und die Freisetzung von Wirkstoffen bzw. deren Trägern gestatten. Beispielsweise Enzyme, welche die Freisetzung vermitteln, biologisch aktive Stoffe aktivieren oder nicht-aktive Beschichtungen binden und zur Exposition von Wirkstoffen führen. Alle hier im speziellen aufgeführten Mechanismen und Eigenschaften sind sowohl auf die primär vorliegende Kohlenstoffschicht anzuwenden, als auch auf darauf zusätzlich aufgebrachte Schichten.

Durch Auftragen obengenannter freisetzungsmodifizierender Polymerschichten und/oder Anpassung der Porenstruktur der kohlenstoffhaltigen Schicht kann die Freisetzung der Wirkstoffe aus dem Implantat in weiten Bereich gesteuert werden. Erreichbare Freisetzungszeiten liegen bei 12 Stunden bis ein oder mehrere Jahre, vorzugsweise 24 Stunden, 48 Stunden, 96 Stunden, 1 Woche, 2 Wochen, 1 Monat, 3 Monate.

Die erfindungsgemäßen Implantate können in besonderen Anwendungen auch mit lebenden Zellen oder Mikroorganismen beladen und damit funktionalisiert werden. Diese können sich in geeignet porösen kohlenstoffhaltigen Schichten ansiedeln, wobei das so besiedelte Implantat dann mit einem geeigneten Membranüberzug versehen werden kann, der für Nährstoffe und von den Zellen oder Mikroorganismen erzeugte Wirkstoffe durchlässig ist, nicht jedoch für die Zellen selbst. So können die Zellen oder Mikroorganismen vom Organismus durch den Membranüberzug versorgt werden.

Auf diese Weise lassen sich unter Anwendung der erfindungsgemäßen Technologie beispielsweise Implantate herstellen, die Insulinproduzierende Zellen enthalten, welche nach Implantierung im Körper in Abhängigkeit vom Glukosespiegel der Umgebung Insulin produzieren und freisetzen.

### Ausführungsbeispiele

1. Verfahren zur Herstellung medizinischer Implantate mit funktionalisierten Oberflächen, umfassend die folgenden Schritte:
   a) Bereitstellung eines medizinischen Implantats mit mindestens einer kohlenstoffhaltigen Schicht auf mindestens einem Teil der Oberfläche des Implantats;
   b) Aktivierung der kohlenstoffhaltigen Schicht durch Schaffung von Porosität;
   c) Funktionalisierung der aktivierten, kohlenstoffhaltigen Schicht.
2. Verfahren nach Ausführungsbeispiel 1,
   **dadurch gekennzeichnet, dass** die kohlenstoffhaltige Schicht ausgewählt ist aus pyrolytisch erzeugtem Kohlenstoff, aufgedampftem Kohlenstoff, mittels CVD-, PVD- oder Sputtern aufgebrachtem Kohlenstoff, Metallcarbiden, Metallcarbonitriden, Metalloxynitriden oder Metalloxycarbiden, sowie beliebigen Kombinationen davon.
3. Verfahren nach Ausführungsbeispiel 1 oder 2,
   **dadurch gekennzeichnet, dass** das Implantat aus einem Material besteht, welches aus Kohlenstoff, Kohlenstoffverbundmaterial, Kohlefasern, Keramik, Glas, Kunststoffe, Metalle, Legierungen, Knochen, Stein, oder Mineralien ausgewählt ist.
4. Verfahren nach einem der vorhergehenden Ausführungsbeispiele,
   **dadurch gekennzeichnet, dass** das Implantat aus medizinischen oder therapeutischen Implantaten wie Gefäßendoprothesen, Stents, Koronarstents, peripheren Stents, chirurgischen oder orthopädischen Implantaten, Knochen- oder Gelenkprothesen, Kunstherzen, künstlichen Herzklappen, subkutanen und/oder intramuskulären Implantaten ausgewählt ist.
5. Verfahren nach einem der vorhergehenden Ausführungsbeispiele,
   **dadurch gekennzeichnet, dass** Aktivierung der kohlenstoffhaltigen Schicht mit geeigneten Oxidationsmitteln und/oder Reduktionsmitteln erfolgt.
6. Verfahren nach einem der vorhergehenden Ausführungsbeispiele,
   **dadurch gekennzeichnet, dass** die kohlenstoffhaltige Schicht durch Oxidation mit Luft, Sauerstoff, Distickstoffmonoxid, und/oder oxidierenden Säuren, gegebenenfalls bei erhöhter Temperatur, aktiviert wird.
7. Verfahren nach einem der vorhergehenden Ausführungsbeispiele,
   **dadurch gekennzeichnet, dass** die Aktivierung durch Abrasion im wässrigen Ultraschallbad unter Zusatz von Tonerde, Silikaten und/oder Aluminaten erfolgt.
8. Verfahren nach einem der vorhergehenden Ausführungsbeispiele,
   **dadurch gekennzeichnet, dass** durch die Aktivierung die kohlenstoffhaltige Schicht porös, vorzugsweise makroporös, mit Porendurchmessern im Bereich von 0,1 bis 1000 µm, wird, ggf. auch durch Vorstrukturierung des Substrats.
9. Verfahren nach einem der vorhergehenden Ausführungsbeispiele,
   **dadurch gekennzeichnet, dass** durch die Aktivierung die kohlenstoffhaltige Schicht nanoporös ist.
10. Verfahren nach einem der vorhergehenden Ausführungsbeispiele,
   **dadurch gekennzeichnet, dass** die aktivierte, poröse kohlenstoffhaltige Schicht mittels CVD und/oder CVI von flüchtigen organischen Stoffen, nachverdichtet und/oder versiegelt wird.
11. Verfahren nach einem der vorhergehenden Ausführungsbeispiele,
   **dadurch gekennzeichnet, dass** die Funktionalisierung der aktivierten, kohlenstoffhaltigen Schicht das Beladen der Schicht mit mindestens einem Stoff ausgewählt aus pharmakologischen Wirkstoffen, Linkern, Mikroorganismen, pflanzliche oder tierische einschließlich menschlicher Zellen oder Zellkulturen und Gewebe, Mineralien, Salze, Metalle, synthetische oder natürliche Polymere, Proteine, Peptide, Aminosäuren, Lösemittel, Ionen, Kationen, insbesondere Metallkationen wie Kobalt-, Nickel-, Kupfer-, Zink-Kationen, Antikörper, Calmodulin, Chitin, Cellulose, Zucker, Aminosäuren, Gluthathion, Streptavidin, Strep-tactin oder andere Mutanten oder S-Protein, Dextrane, sowie sowie deren Derivate, Mischungen und Kombinationen umfasst.
12. Verfahren nach einem der vorhergehenden Ausführungsbeispiele,
   **dadurch gekennzeichnet, dass** die Funktionalisierung durch die Adsorption von mit Affinitätsanhängseln (affinity tags) korrespondierenden Substanzen in und/oder an der kohlenstoffhaltigen Schicht erfolgt, wobei die korrespondierenden Substanzen so gewählt sind, dass sie mit den Affinitätsanhängseln eine Bindung eingehen können.
13. Verfahren nach Ausführungsbeispiel 11 oder 12,
   **dadurch gekennzeichnet, dass** der oder die Stoffe bzw. Substanzen durch Adsorption, Absorption, Physisorption, Chemisorption, elektrostatische, kovalente Bindung, oder nicht-kovalente Bindung auf der kohlenstoffhaltigen Schicht aufgebracht bzw. immobilisiert wird/werden.
14. Verfahren nach Ausführungsbeispiel 11,
   **dadurch gekennzeichnet, dass** der mindestens eine Stoff im wesentlichen permanent auf der(den) kohlenstoffhaltigen Schicht(en) immobilisiert ist.
15. Verfahren nach Ausführungsbeispiel 11,
   **dadurch gekennzeichnet, dass** der mindestens eine auf die kohlenstoffhaltige Schicht aufgebrachte Stoff, insbesondere pharmakologische Wirkstoff, aus der Schicht kontrolliert freisetzbar ist.
16. Verfahren nach Ausführungsbeispiel 15,
   **dadurch gekennzeichnet, dass** die pharmakologisch wirksamen Stoffe in Microcapsules, Liposomen, Nanocapsules, Nanopartikeln, Micellen, synthetischen Phospholipide, Gas-Dispersionen, Emulsionen, Mikroemulsionen, oder Nanospheres inkorporiert werden, die in den Poren oder an der Oberfläche der kohlenstoffhaltigen Schicht adsorbiert werden und dann therapeutisch freisetzbar sind.
17. Verfahren nach einem der Ausführungsbeispiel 14 bis 16,
   **dadurch gekennzeichnet, dass** ferner eine die Freisetzung des Wirkstoffs beeinflussende Beschichtung ausgewählt aus pH-sensitiven und/oder Temperatursensitiven Polymeren und/oder biologisch aktiven Barrieren wie z.B. Enzymen aufgebracht wird.
18. Verfahren nach einem der vorhergehenden Ausführungsbeispiele,
   **dadurch gekennzeichnet, dass** die Funktionalisierung das Aufbringen von biologisch abbaubaren bzw. resorbierbaren Polymeren wie Kollagen, Albumin, Gelatine, Hyaluronsäure, Stärke, Cellulosen wie Methylcellulose, Hydroxypropylcellulose, Hydroxypropylmethylcellulose, Carboxymethylcellulose-Phtalat; Kasein, Dextrane, Polysaccharide, Fibrinogen, Poly(D,L-Lactide), Poly(D,L-Lactide-Co-Glycolide), Poly(Glycolide), Poly(Hydroxybutylate), Poly(Alkylcarbonate), Poly(Orthoester), Polyester, Poly(Hydroxyvalerinsäure), Polydioxanone, Poly(Ethylenterephtalate), Poly(malatsäure), Poly(Tartronsäure), Polyanhydride, Polyphosphazene, Poly(Aminosäuren), und deren Co-Polymeren, umfasst.
19. Verfahren nach einem der vorhergehenden Ausführungsbeispiele,
   **dadurch gekennzeichnet, dass** die Funktionalisierung das Aufbringen von nicht biologisch abbaubaren bzw. resorbierbaren Polymeren wie Poly(Ethylen-Vinylacetate), Silicone, Acrylpolymere wie Polyacrylsäure, Polymethylacrylsäure, Polyacrylcyanoacrylat; Polyethylene, Polypropylene, Polyamide, Polyurethane, Poly(Ester-Urethane), Poly(Ether-Urethane), Poly(Ester-Harnstoffe), Polyether (Poly(Ethylenoxid), Poly(Propylenoxid), Pluronics, Poly(Tetramethylenglycol); Vinylpolymere wie Polyvinylpyrrolidone, Poly(Vinylalkohole), oder Poly(vinylacetat-phatalat), sowie deren Copolymeren, umfasst.
20. Implantat mit funktionalisierter Oberfläche, herstellbar nach einem der vorhergehenden Ausführungsbeispiele.
21. Implantat nach Ausführungsbeispiel 20,
   **dadurch gekennzeichnet, dass** es aus Metallen wie rostfreier Stahl, Titan, Tantal, Platin, Gold, Palladium, Legierungen, insbesondere Formgedächtnislegierungen wie Nitinol, oder Nickel-Titan Legierung, oder aus Kohlefasern, Vollkarbonmaterial, oder Kohlenstoffkompositen besteht.
22. Implantat nach Anspruch einem der Ausführungsbeispiele 20 oder 21, umfassend mehrere, gegebenenfalls mit Wirkstoff beladene, kohlenstoffhaltige Schichten.
23. Vorrichtung nach einem der Ausführungsbeispiele 20 bis 22,
   ferner umfassend anionische oder kationische oder amphotere Beschichtungen, ausgewählt aus Alginat, Carrageenan, Carboxymethylcellulose; Poly(meth)acrylaten, Chitosan, Poly-L-Lysine; und/oder Phoshporylcholin.
24. Wirkstoffbeschichteter Stent nach einem der Ausführungsbeispiele 20 bis 23.
25. Wirkstoffbeschichtete Herzklappe nach einem der Ausführungsbeispiele 20 bis 23.
26. Implantat nach einem der Ausführungsbeispiele 20 bis 23, in Form einer orthopädischen Knochen- oder Gelenksprothese, eines Knochensubstituts oder eines Wirbelkörperersatzmittels im Brust- oder Lendenbereich der Wirbelsäule.
27. Subkutan und/oder intramuskulär einsetzbares Wirkstoffdepot mit kontrollierter Freisetzung, nach einem der Ausführungsbeispiele 20 bis 23.
28. Implantat nach einem der Ausführungsbeispiele 20 bis 27, umfassend aufgebrachte bzw. inkorporierte Mikroorganismen, virale Vektoren oder Zellen oder Gewebe.
29. Verwendung eines Implantats nach Ausführungsbeispiel 28, zur Erzeugung einer therapeutischen Wirkung oder zur Erhöhung der Bioverträglichkeit des Implantats nach dem Einsetzen des Implantats in den menschlichen Körper.

## Patentansprüche

1. Medizinisches Implantat mit einer funktionalisierten Oberfläche, wobei das Implantat eine kohlenstoffhaltige Schicht auf mindestens einem Teil der Oberfläche des Implantats aufweist; die kohlenstoffhaltige Schicht durch Schaffung von Porosität aktiviert ist; und die aktivierte, poröse, kohlenstoffhaltige Schicht eine Funktionalisierung aufweist.

2. Implantat nach Anspruch 1,
**dadurch gekennzeichnet, dass** die kohlenstoffhaltige Schicht ausgewählt ist aus pyrolytisch erzeugtem Kohlenstoff, glasartig amorphem Kohlenstoff, aufgedampftem Kohlenstoff, mittels CVD-, PVD- oder Sputtern aufgebrachtem Kohlenstoff, diamantartigem Kohlenstoff, graphitischem Kohlenstoff, Metallcarbiden, Metallcarbonitriden, Metalloxynitriden oder Metalloxycarbiden, sowie beliebigen Kombinationen davon.

3. Implantat nach Anspruch 1 oder 2,
**dadurch gekennzeichnet, dass** das Implantat aus einem Material besteht, welches aus Kohlenstoff, Kohlenstoffverbundmaterial, Kohlefasern, Keramik, Glas, Kunststoffe, Metalle, Legierungen, Knochen, Stein, oder Mineralien ausgewählt ist.

4. Implantat nach Anspruch 3,
**dadurch gekennzeichnet, dass** es aus Metallen wie rostfreier Stahl, Titan, Tantal, Platin, Gold, Palladium, Legierungen, insbesondere Formgedächtnislegierungen wie Nitinol, oder Nickel-Titan Legierung, oder aus Kohlefasern, Vollkarbonmaterial, oder Kohlenstoffkompositen besteht.

5. Implantat nach Anspruch 4,
**dadurch gekennzeichnet, dass** es aus rostfreiem Stahl, bevorzugt Fe-18Cr-14Ni-2.5Mo, Fe-21Cr-10Ni-3.5Mn-2.5Mo, Fe-22Cr-13Ni-5Mn, Fe-23Mn-21Cr-1Mo-1N; aus Kobaltlegierungen, bevorzugt Co-20Cr-15W-10Ni, Co-20Cr-35Ni-10Mo, Co-20Cr-16Ni-16Fe-7Mo; aus Titanlegierungen, bevorzugt CP-Titanium, Ti-6Al-4V, Ti-6Al-7Nb, Ti-15Mo; aus Edelmetalllegierungen, bevorzugt iridiumhaltige Legierungen, Nitinollegierungen und Magnesiumlegierungen.

6. Implantat nach einem der vorhergehenden Ansprüche,
**dadurch gekennzeichnet, dass** das Implantat aus medizinischen oder therapeutischen Implantaten, bevorzugt aus Gefäßendoprothesen, Stents, Koronarstents, peripheren Stents, chirurgischen oder orthopädischen Implantaten, Knochen- oder Gelenkprothesen, Kunstherzen, künstlichen Herzklappen, subkutanen und/oder intramuskulären Implantaten, ausgewählt ist.

7. Implantat nach einem der vorhergehenden Ansprüche,
**dadurch gekennzeichnet, dass** die Porosität der kohlenstoffhaltigen Schicht makroporös ist, bevorzugt mit einem Porendurchmesser im Bereich von 0,1 µm bis 1000 µm, weiter bevorzugt im Bereich von 1 µm bis 400 µm.

8. Implantat nach einem der vorhergehenden Ansprüche,
**dadurch gekennzeichnet, dass** die Porosität der kohlenstoffhaltigen Schicht nanoporös ist, bevorzugt mit einem Porendurchmesser von 1 nm bis 1000 nm, weiter bevorzugt von 5 nm bis 900 nm.

9. Implantat nach einem der vorhergehenden Ansprüche,
**dadurch gekennzeichnet, dass** die aktivierte, poröse kohlenstoffhaltige Schicht mittels CVD und/oder CVI von flüchtigen organischen Stoffen nachverdichtet und/oder versiegelt ist.

10. Implantat nach einem der vorhergehenden Ansprüche,
**dadurch gekennzeichnet, dass** die aktivierte, poröse kohlenstoffhaltige Schicht mit mindestens einem Stoff, ausgewählt aus pharmakologischen Wirkstoffen, Linkern, Mikroorganismen, pflanzliche oder tierische einschließlich menschlicher Zellen oder Zellkulturen und Gewebe, Mineralien, Salze, Metalle, synthetische oder natürliche Polymere, Proteine, Peptide, Aminosäuren, Lösemittel, Ionen, Kationen, insbesondere Metallkationen wie Kobalt-, Nickel-, Kupfer-, Zink-Kationen, Antikörper, Calmodulin, Chitin, Cellulose, Zucker, Aminosäuren, Gluthathion, Streptavidin, Strep-tactin oder andere Mutanten oder S-Protein, Dextrane, sowie deren Derivate, Mischungen und Kombinationen, beladen ist.

11. Implantat nach Anspruch 10,
**dadurch gekennzeichnet, dass** die aktivierte, poröse kohlenstoffhaltige Schicht mit mindestens einem pharmakologischen Wirkstoff beladen ist, bevorzugt Paclitaxel, besonders bevorzugt in einer Beladungsmenge von 0,5 bis 3,0 µg/mm².

12. Implantat nach Anspruch 10 oder 11,
**dadurch gekennzeichnet, dass** der mindestens eine auf die kohlenstoffhaltige Schicht aufgebrachte Stoff, insbesondere ein pharmakologischer Wirkstoff, aus der Schicht kontrolliert freisetzbar ist.

13. Implantat nach Anspruch 10 bis 12,
**dadurch gekennzeichnet, dass** ferner eine die Freisetzung des Wirkstoffs beeinflussende Beschichtung, ausgewählt aus pH-sensitiven und/oder Temperatursensitiven Polymeren und/oder biologisch aktiven Barrieren, bevorzugt Enzymen, aufgebracht ist.

14. Implantat nach Anspruch 12,
**dadurch gekennzeichnet, dass** die die Freisetzung des Wirkstoffs beeinflussende ausgewählt ist aus Poly(Acrylsäure) und deren Derivaten, bevorzugt Poly(Aminocarboxylsäure), Poly(Acrylsäure), Poly(Methyl-Acrylsäure) und deren Co-Polymeren; Polysacchariden, bevorzugt Celluloseacetat-Phtalat, Hydroxypropylmethylcellulose-Phtalat, Hydroxypropylmethylcellulose-Succinat, Celluloseacetat-Trimellitat und/oder Chitosan; Poly(N-Isopropylacrylamid-Co-Natrium-Acrylat-Co-n-N-Alkylacrylamid), Poly(N-Methyl-N-n-propylacrylamid), Poly(N-Methyl-N-Isopropylacrylamid), Poly(N-n-Propylmethacrylamid), Poly(N-Isopropylacrylamid), Poly(N,n-Diethylacrylamid), Poly(N-Isopropylmethacrylamid), Poly(N-Cyclopropylacrylamid), Poly(N-Ethylacrylamid), Poly(N-Ethylmethyacrylamid), Poly(N-Methyl-N-Ethylacrylamid), Poly(N-Cyclopropylacrylamid), Hydroxypropyl-Cellulose, Methyl-Cellulose, Hydroxypropylmethyl-Cellulose, Ethylhydroxyethyl-Cellulose und Pluronics.

15. Implantat nach einem der vorhergehenden Ansprüche,
**dadurch gekennzeichnet, dass** das Implantat ein wirkstoffbeschichteter Stent, bevorzugt ein wirkstoffbeschichteter Koronarstent ist.
